(19)

Europäisches
Patentamt
European
Patent Office
Office européen
des brevets

(11) **EP 4 462 121 A2**

(12) **EUROPEAN PATENT APPLICATION**

(43) Date of publication:
**13.11.2024   Bulletin 2024/46**

(21) Application number: **24188930.2**

(22) Date of filing: **09.09.2016**

(51) International Patent Classification (IPC):
**G01N 33/53** *(2006.01)*

(52) Cooperative Patent Classification (CPC):
**C07K 16/18; A61P 1/00; A61P 3/10; A61P 7/00;**
**A61P 9/00; A61P 11/00; A61P 13/12; A61P 17/00;**
**A61P 19/00; A61P 21/00; A61P 25/00;**
**A61P 27/00; A61P 29/00; A61P 37/02;**
**C07K 16/40;**                                    (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:   **11.09.2015   US 201562217177 P**

(62) Document number(s) of the earlier application(s) in
accordance with Art. 76 EPC:
**16775899.4 / 3 328 885**

(71) Applicant: **Alexion Pharmaceuticals, Inc.**
**New Haven, CT 06510 (US)**

(72) Inventors:
  • **ANDRIEN, Bruce**
    **Guilford, CT 06437 (US)**
  • **HUNTER, Jeffrey**
    **New Haven, CT 06510 (US)**

  • **MALANSON, Hunter**
    **New Haven, CT 06510 (US)**

(74) Representative: **J A Kemp LLP**
**80 Turnmill Street**
**London EC1M 5QU (GB)**

Remarks:
  •The complete document including Reference
  Table(s) and the Sequence Listing(s) can be
  downloaded from the EPO website
  •This application was filed on 16-07-2024 as a
  divisional application to the application mentioned
  under INID code 62.
  •Claims filed after the date of filing of the
  application/after the date of receipt of the divisional
  application (Rule 68(4) EPC).

(54)     **RECOMBINANT GLYCOSYLATED ECULIZUMAB AND ECULIZUMAB VARIANTS**

(57)     The present disclosure relates to, *inter alia,* a recombinant eculizumab protein or recombinant eculizumab variant protein having specific glycosylation patterns. The present disclosure relates to, *inter alia,* a recombinant eculizumab protein or a recombinant eculizumab variant protein made from CHO cells. The present disclosure also relates to methods for the use of these proteins.

**EP 4 462 121 A2**

(52) Cooperative Patent Classification (CPC): (Cont.)
A61K 2039/505; C07K 2317/14; C07K 2317/24;
C07K 2317/41; C07K 2317/76; C07K 2317/94

## Description

### INCORPORATION OF SEQUENCE LISTING

The instant application contains a Sequence Listing which has been submitted electronically in ASCII format and is hereby incorporated by reference in its entirety. Said ASCII copy, created on September 6, 2016, is named ALXN305PCT_SL.txt and is 16,120 bytes in size.

### TECHNICAL FIELD

This application relates to the fields of immunology and biotechnology.

### BACKGROUND

Eculizumab is a humanized anti-human C5 monoclonal antibody (Alexion Pharmaceuticals, Inc.), with a human IgG2/IgG4 hybrid constant region, so as to reduce the potential to elicit proinflammatory responses. Eculizumab has the trade name Soliris® and is currently approved for treating paroxysmal nocturnal hemoglobinuria ("PNH") and atypical hemolytic uremic syndrome ("aHUS"). Eculizumab has also been shown in a recent clinical trial to be effective for patients with Shiga-toxin-producing E. *coli* hemolytic uremic syndrome ("STEC-HUS"). *See* Alexion press release, "New Clinical Trial Data Show Substantial Improvement with Eculizumab (Soliris®) in Patients with STEC-HUS," Saturday, November 3, 2012.

Soliris® is a recombinant protein made from NS0 cells, a murine myeloma cell line, in a media that contains bovine serum albumin (BSA). A recombinant Eculizumab made from NS0 cells has a specific glycosylation pattern, which may have an impact on the function of the protein. The current process uses a 10,000 and 20,000L stirred tank bioreactor with media that contains animal material (bovine serum albumin, or BSA). There is also some lot-to-lot variability.

### SUMMARY

This disclosure provides a recombinant eculizumab protein or a recombinant eculizumab variant protein having one or more of the structural features of: less than 0.1 mmol/mol of N-Glycolylneuraminic acid (NGNA); less than 0.02 nmol/mg protein of N-acetylgalactose amine (GalNAc); or a percentage of neutral glycans that is above about 99% of total glycans.

In another aspect, a Chinese Hamster Ovary ("CHO") cell is provided; this cell bears an expression vector capable of expressing (or that expresses) an eculizumab protein or an eculizumab variant protein in that CHO cell.

In another aspect, a recombinant eculizumab protein or a recombinant eculizumab variant protein is provided; the recombinant eculizumab protein or the recombinant eculizumab variant protein is produced in a Chinese Hamster Ovary ("CHO") cell bearing an expression vector capable of expressing (or that expresses) the eculizumab protein or the eculizumab variant protein in the CHO cell.

In another aspect, a method is provided of inhibiting formation of terminal complement in a biological sample, the method comprising contacting a biological sample with a therapeutic agent in an amount effective to inhibit terminal complement in the biological sample, wherein the biological sample is capable of terminal complement production in the absence of the therapeutic agent and wherein the therapeutic agent is the recombinant eculizumab protein or the recombinant eculizumab variant protein disclosed herein.

In another aspect, a method is provided of treating a patient in need of treatment with eculizumab or an eculizumab variant, comprising administering to said patient the recombinant eculizumab protein or the recombinant eculizumab variant protein disclosed herein.

Numerous other aspects are provided in accordance with these and other aspects of the disclosure. Other features and aspects of the present disclosure will become more fully apparent from the detailed description and the appended claims.

### DETAILED DESCRIPION

### Definitions

As used herein, the word "a" or "plurality" before a noun represents one or more of the particular noun. For example, the phrase "a mammalian cell" represents "one or more mammalian cells."

For the terms "for example" and "such as," and grammatical equivalences thereof, the phrase "and without limitation" is understood to follow unless explicitly stated otherwise. As used herein, the term "about" is meant to account for variations due to experimental error. All measurements reported herein are understood to be modified by the term "about", whether or not the term is explicitly used, unless explicitly stated otherwise.

The term "recombinant protein" is known in the art. A recombinant protein can be a glycoprotein. For example, recombinant eculizumab or a recombinant eculizumab variant made in a CHO cell is glycosylated, with the sugar moieties covalently attached on the protein, and is a glycoprotein. Briefly, the term "recombinant protein" can refer to a protein that can be manufactured using a cell culture system. The cells in the cell culture system can be derived from, for example, a mammalian cell, including a human cell, a CHO cell, an insect cell, a yeast cell, or a bacterial cell. In general, the cells in the cell culture contain an introduced nucleic acid encoding the recombinant protein of interest (which nucleic acid can be borne on a vector, such as a plasmid vector). The nucleic acid encoding the recombinant protein can also contain a heterologous promoter operably linked to a nucleic acid encoding the protein.

The term "mammalian cell" is known in the art and can refer to any cell from or derived from any mammal including, for example, a human, a hamster, a mouse, a green monkey, a rat, a pig, a cow, a hamster, or a rabbit. The mammalian cell can be an immortalized cell, a differentiated cell, or an undifferentiated cell.

The term "liquid culture medium" is known in the art. Briefly, it means a fluid that includes sufficient nutrients to allow a mammalian cell to grow or proliferate *in vitro.* For example, a liquid culture medium can include one or more of: amino acids (e.g., 20 amino acids), a purine (e.g., hypoxanthine), a pyrimidine (e.g., thymidine), choline, inositol, thiamine, folic acid, biotin, calcium, niacinamide, pyridoxine, riboflavin, thymidine, cyanocobalamin, pyruvate, lipoic acid, magnesium, glucose, sodium, potassium, iron, copper, zinc, and sodium bicarbonate. In some embodiments, a liquid culture medium can include serum from a mammal. In some embodiments, a liquid culture medium does not include serum or another extract from a mammal (a defined liquid culture medium). In some embodiments, a liquid culture medium can include trace metals, a mammalian growth hormone, and/or a mammalian growth factor. Another example of liquid culture medium is minimal medium (e.g., a medium that includes only inorganic salts, a carbon source, and water). Examples of liquid culture medium are known in the art and are commercially available. A liquid culture medium may include any density of mammalian cells. For example, a volume of liquid culture medium removed from a bioreactor can be substantially free of mammalian cells.

The term "serum-free liquid culture medium" is known in the art. Briefly, it means a liquid culture medium that does not include a mammalian serum.

The term "serum-containing liquid culture medium" is known in the art. Briefly, it means a liquid culture medium that includes a mammalian serum (such as BSA).

The term "chemically-defined liquid culture medium" is a term of art and means a liquid culture medium in which all of the chemical components are known. For example, a chemically-defined liquid culture medium does not include fetal bovine serum, bovine serum albumin, or human serum albumin, as these preparations typically include a complex mix of albumins and lipids.

The term "protein-free liquid culture medium" is known in the art and it means a liquid culture medium that does not include any protein (e.g., any detectable protein).

The term "continuous process" is known in the art and means a process which continuously feeds fluid through at least a part of a system for producing a drug substance (e.g., a drug substance containing a recombinant protein) from a culture medium containing the recombinant protein (e.g., any of the recombinant proteins described herein). For example, a liquid culture medium that includes a recombinant therapeutic protein (e.g., recombinant human $\alpha$-galactosidase-A protein) is continuously fed into the system while it is in operation and a therapeutic protein drug substance is fed out of the system. Non-limiting examples of such systems that can be used to perform a continuous process are described in U.S. Provisional Patent Application Nos. 61/856,930 and 61/775,060.

The term "perfusion culturing" is known in the art and means culturing a plurality of cells (e.g., mammalian cells) in a first liquid culture medium, wherein the culturing includes periodic or continuous removal of the first liquid culture medium and at the same time or shortly after adding substantially the same volume of a second liquid culture medium to a container (e.g., a bioreactor). In some examples, there is an incremental change (e.g., increase or decrease) in the volume of the first liquid culture medium removed and added over incremental periods (e.g., an about 24-hour period, a period of between about 1 minute and about 24-hours, or a period of greater than 24 hours) during the culturing period (e.g., the culture medium refeed rate on a daily basis). The fraction of media removed and replaced each day can vary depending on the particular cells being cultured, the initial seeding density, and the cell density at a particular time. "RV" or "reactor volume" means the volume of the culture medium present at the beginning of the culturing process (e.g., the total volume of the culture medium present after seeding). Bioreactors that can be used to perform perfusion culturing are known in the art. A skilled artisan will appreciate that a bioreactor can be adapted to be used in perfusion culturing (e.g., adapted to be a perfusion bioreactor).

The term "fed-batch culturing" is a term of art and means culturing a plurality of cells (e.g., mammalian cells) in a first liquid culture medium, wherein the culturing of the cells present in a container (e.g., a bioreactor) includes the periodic or continuous addition of a second liquid culture medium to the first liquid culture medium without substantial or significant removal of the first liquid culture medium or second liquid culture medium from the cell culture. The second liquid culture medium can be the same as the first liquid culture medium. In some examples of fed-batch culture, the second liquid culture medium is a concentrated form of the first liquid culture medium. In some examples of fed-batch culture, the second liquid culture medium is added as a dry powder. A skilled artisan will appreciate that a bioreactor can be adapted to be used in fed-batch culturing (e.g., adapted to be a fed-batch bioreactor).

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

In certain aspects, a recombinant eculizumab protein or a recombinant eculizumab variant protein is provided. The protein has one or more of the structural features of: less than 0.1 mmol/mol of N-Glycolylneuraminic acid (NGNA); less than 0.02 nmol/mg protein of N-acetylgalactose amine (GalNAc); or a percentage of neutral glycans that is above about 99% of total glycans. In certain embodiments, the protein has one or more of the structural features of: no detectable N-Glycolylneuraminic acid (NGNA); no detectable N-acetylgalactose amine (GalNAc); or a percentage of neutral glycans that is above about 99% of total glycans.

In other aspects, a recombinant eculizumab protein or a recombinant eculizumab variant protein is provided; the protein is produced in a Chinese Hamster Ovary ("CHO") cell bearing an expression vector capable of expressing (or that expresses) said eculizumab protein or said eculizumab variant protein in said CHO cell. The expression vector can be either constitutive or inducible for expressing the eculizumab protein or the eculizumab variant protein in the CHO cell. In certain embodiments, the recombinant eculizumab protein or the recombinant eculizumab variant protein has one or both of the structural features of: less than 0.1 mmol/mol of N-Glycolylneuraminic acid (NGNA); or a percentage of neutral glycans that is above about 99% of total glycans.

In other aspects, a Chinese Hamster Ovary ("CHO") cell is provided, the cell bearing an expression vector capable of expressing (or that expresses) an eculizumab protein or an eculizumab variant protein in said CHO cell. The expression vector can be either constitutive or inducible for expressing the eculizumab protein or the eculizumab variant protein in the CHO cell.

In other aspects, a pharmaceutical composition is provided, the composition comprising the recombinant eculizumab protein or the recombinant eculizumab variant protein disclosed herein, and a pharmaceutically acceptable carrier. The pharmaceutical composition can be formulated for intravenous, intraarterial, intramuscular, intradermal, subcutaneous, or intraperitoneal administration. In certain embodiments, the pharmaceutical composition comprises recombinant eculizumab protein or recombinant eculizumab variant protein at least 10 mg/mL, but less than or equal to 100 mg/mL. In certain embodiments, the pharmaceutical composition comprises recombinant eculizumab protein or recombinant eculizumab variant protein at greater than 100 mg/mL.

In yet other aspects, a method for inhibiting formation of terminal complement in a biological sample is provided, the method comprising contacting a biological sample with a therapeutic agent in an amount effective to inhibit terminal complement in the biological sample, wherein the biological sample is capable of terminal complement production in the absence of the therapeutic agent and wherein the therapeutic agent is the recombinant eculizumab protein or the recombinant eculizumab variant protein disclosed herein.

A method of treating a patient in need of treatment with eculizumab or an eculizumab variant, comprising administering to said patient the recombinant eculizumab protein or the recombinant eculizumab variant protein disclosed herein.

A CHO cell line is well known in the art. Reference to a CHO cell or a CHO cell line refers to any CHO cell or CHO cell line, and any derivative of a CHO cell, including CHO mutant cells such as, for example, CHO glycosylation mutants. See, e.g., Jayapal K. P., Wlaschin K. F., Yap M. G. S., Hu W-S.,

(2007), "Recombinant protein therapeutics from CHO cells - 20 years and counting." Chem. Eng. Prog. 103 (10): 40-47; see also North et al., J. Biol Chem Vol. 285, No. 8, pp. 5759-5775, February 19, 2010.

The CHO cell can be cultured in any medium by any means. In certain embodiments, the CHO cell is cultured in a liquid culture medium. In certain embodiments, the CHO cell is cultured in a liquid culture medium without any animal derived raw materials, such as, for example, BSA. In certain embodiments, the CHO cell is cultured in a chemically-defined liquid culture medium or a protein-free liquid culture medium. In certain embodiments, the CHO cell is cultured by a continuous process. In certain embodiments, the CHO cells are cultured by fed-batch culturing or by perfusion culturing. The CHO cells can be cultured in monolayer cultures and in suspension cultures.

The recombinant eculizumab protein or a recombinant eculizumab variant protein is not a protein that is normally produced by a CHO cell, as eculizumab and its variant are humanized anti-human-C5 antibodies; whereas the CHO cells are hamster cells.

Eculizumab is a humanized anti-human C5 monoclonal antibody (Alexion Pharmaceuticals, Inc.), with a human IgG2/IgG4 hybrid constant region, so as to reduce the potential to elicit proinflammatory responses. Eculizumab has the trade name Soliris® and is currently approved for treating paroxysmal nocturnal hemoglobinuria ("PNH") and atypical hemolytic uremic syndrome ("aHUS"). Paroxysmal nocturnal hemoglobinuria is a form of hemolytic anemia, intravascular hemolysis being a prominent feature due to the absence of the complement regulatory protein CD59 and CD55. CD59, for example, functions to block the formation of the terminal complement complex. AHUS involves chronic uncontrolled complement activation, resulting in, *inter alia,* inhibition of thrombolitic microangiopathy, the formation of blood clots in small blood vessels throughout the body, and acute renal failure. Eculizumab specifically binds to human C5 protein and blocks the formation of the generation of the potent proinflammatory protein C5a. Eculizumab further blocks the formation of the terminal complement complex. Eculizumab treatment reduces intravascular hemolysis in patients with PNH and decreases complement levels in aHUS. *See, e.g.,* Hillmen et al., N Engl J Med 2004; 350:552-9; Rother et al., Nature Biotechnology 2007; 25(11): 1256-1264; Hillmen et al., N Engl J Med 2006, 355;12, 1233-1243; Zuber et al., Nature Reviews Nephrology 8, 643-657 (2012) | doi:10.1038/nrneph.2012.214; U.S. Patent Publication Number 2012/0237515, and U.S. Patent Number 6,355,245. Eculizumab has also been shown in a recent clinical trial to be effective for patients with Shiga-toxin-producing E. *coli* hemolytic uremic syndrome ("STEC-HUS"). *See* Alexion press release, "New Clinical Trial Data Show Substantial Improvement with Eculizumab (Soliris®) in Patients with STEC-HUS," Saturday, November 3, 2012. STEC-HUS is characterized by systemic complement-mediated thrombotic microangiopathy and acute vital organ damage. Eculizumab administration to these patients resulted in rapid and sustained improvement in thrombotic microangiopathy and improvements in systemic organ complications. As can be seen, PNH, aHUS, and STEC-HUS are all diseases relating to inappropriate complement activation. See, *e.g.,* Noris et al., Nat Rev Nephrol. 2012 Nov;8(11):622-33. doi: 10.1038/nrneph.2012.195. Epub 2012 Sep 18; Hillmen et al., N Engl J Med 2004; 350:6, 552-9; Rother et al., Nature Biotechnology 2007; 25(11): 1256-1264; Hillmen et al., N Engl J Med 2006, 355;12, 1233-1243; Zuber et al., Nature Reviews Nephrology 8, 643-657 (2012) | doi:10.1038/nrneph.2012.214.

SEQ ID NO:1 depicts the entire heavy chain of eculizumab; SEQ ID NO:2 depicts the entire light chain of eculizumab; SEQ ID NOs:5-7 depict, respectively, CDR1-3 of the heavy chain of eculizumab; SEQ ID NOs:8-10 depict, respectively, CDR1-3 of the light chain of eculizumab; SEQ ID NO:11 depicts the variable region of the heavy chain of eculizumab; and SEQ ID NO:12 depicts the variable region of the light chain of Eculizumab.

In certain embodiments, the anti-C5 antibody is a variant derived from eculizumab, having one or more improved properties (e.g., improved pharmacokinetic properties) relative to eculizumab. The variant eculizumab antibody (also referred to herein as an eculizumab variant, a variant eculizumab, or the like) or C5-binding fragment thereof is one that: (a) binds to complement component C5; (b) inhibits the generation of C5a; and can further inhibit the cleavage of C5 into fragments C5a and C5b. The variant eculizumab antibody can have a serum half-life in a human that is greater than, or at least, 10 (e.g., greater than, or at least, 11, 12, 13, 14, 15, 16, 17, 18, 19, 20, 21, 22, 23, 24, 25, 26, 27, 28, 29, 30, 31, 32, 33 or 34) days. Such variant eculizumab antibodies are described in U.S. Patent Number 9,079,949. Methods of making eculizumab variants, by, for example, recombinant DNA technology, are well known in the art.

In certain embodiments, the eculizumab variant antibody is an antibody defined by the sequences depicted in SEQ ID NO:3 (heavy chain) and SEQ ID NO:4 (light chain), or an antigen-binding fragment thereof. This antibody binds to human C5 and inhibits the formation of C5a, as well as the cleavage of C5 to fragments C5a and C5b, and thus preventing the formation of terminal complement complex.

The terms "eculizumab" and "eculizumab variant" include a C5-binding polypeptide that is not a whole antibody. In some embodiments, a C5-binding polypeptide is a single chain antibody version of eculizumab or an eculizumab variant.

The terms "eculizumab" and "eculizumab variant" include C5-binding polypeptide that comprise, or can consist of, the amino acid sequence depicted in SEQ ID NO:1 and SEQ ID NO:2, or SEQ ID NO: 3 and SEQ ID NO: 4, or an antigen binding fragment of any of the above; the polypeptide can comprise one or more of the amino acid sequence depicted in SEQ ID NOs:5-11.

The terms "eculizumab" and "eculizumab variant" include a fusion protein comprising eculizumab or an eculizumab variant. The fusion protein can be constructed recombinantly such that the fusion protein is expressed from a nucleic acid that encodes the fusion protein. The fusion protein can comprise one or more segments that are heterologous to the eculizumab or the eculizumab variant. The heterologous sequence can be any suitable sequence, such as, for example, an antigenic tag (e.g., FLAG, polyhistidine, hemagglutinin ("HA"), glutathione-S-transferase ("GST"), or maltose-binding protein ("MBP")). Heterologous sequences can also be proteins useful as diagnostic or detectable markers, for example, luciferase, green fluorescent protein ("GFP"), or chloramphenicol acetyl transferase ("CAT"). In some embodiments, the heterologous sequence can be a targeting moiety that targets the C5-binding segment to a cell, tissue, or microenvironment of interest. In some embodiments, the targeting moiety is a soluble form of a human complement receptor (e.g., human complement receptor 2) or an antibody (e.g., a single chain antibody) that binds to C3b or C3d. In some embodiments, the targeting moiety is an antibody that binds to a tissue-specific antigen, such as a kidney-specific antigen. Methods of constructing such fusion proteins, such as by recombinant DNA technology, are well known in the art.

Methods for generating fusion proteins (e.g., fusion proteins containing a C5-binding polypeptide and a soluble form of human CR1 or human CR2), including recombinant DNA technology, are known in the art and described in, e.g., U.S. patent no. 6,897,290; U.S. patent application publication no. 2005265995; and Song et al. (2003) J Clin Invest 11(12):1875-1885.

In some embodiments, the terms "eculizumab" and "eculizumab variant" include a C5-binding polypeptide that is a bispecific antibody. Methods for producing a bispecific antibody are also known in the art. A wide variety of bispecific antibody formats are known in the art of antibody engineering and methods for making the bispecific antibodies are well within the purview of those skilled in the art. *See, e.g.,* Suresh et al. (1986) Methods in Enzymology 121:210; PCT Publication No. WO 96/27011; Brennan et al. (1985) Science 229:81; Shalaby et al., J. Exp. Med. (1992) 175:217-225; Kostelny et al. (1992) J Immunol 148(5):1547-1553; Hollinger et al. (1993) Proc Natl Acad Sci USA 90:6444-6448; Gruber et al. (1994) J Immunol 152:5368; and Tutt et al. (1991) J Immunol 147:60. Any other antibody can be part of the bispecific antibody with eculizumab or an eculizumab variant. Antibody to C3b, C3d, or a lung-specific antigen, an eye-specific antigen, and a kidney-specific antigen are but a few examples.

Bispecific antibodies also include cross-linked or heteroconjugate antibodies. Heteroconjugate antibodies may be made using any convenient cross-linking methods. Suitable cross-linking agents are well known in the art, and are disclosed in U.S. Pat. No. 4,676,980, along with a number of cross-linking techniques. U.S. Patent No. 5,534,254 describes several different types of bispecific antibodies including, e.g., single chain Fv fragments linked together by peptide couplers, chelating agents, or chemical or disulfide couplings. In another example, Segal and Bast [(1995) Curr Protocols Immunol Suppl. 14:2.13.1-2.13.16] describes methods for chemically cross-linking two monospecific antibodies to thus form a bispecific antibody.

The bispecific antibody can be a tandem single chain (sc) Fv fragment, which contains two different scFv fragments covalently tethered together by a linker (e.g., a polypeptide linker). *See, e.g.,* Ren-Heidenreich et al. (2004) Cancer 100:1095-1103 and Korn et al. (2004) J Gene Med 6:642-651.

In some embodiments, the linker can contain, or be, all or part of a heavy chain polypeptide constant region such as a CH1 domain as described in, e.g., Grosse-Hovest et al. (2004) Proc Natl Acad Sci USA 101:6858-6863. In some embodiments, the two antibody fragments can be covalently tethered together by way of a polyglycine-serine or polyserine-glycine linker as described in, e.g., U.S. patent nos. 7,112,324 and 5,525,491, respectively. *See also* U.S. patent no. 5,258,498. Methods for generating bispecific tandem scFv antibodies are described in, e.g., Maletz et al. (2001) Int J Cancer 93:409-416; Hayden et al. (1994) Ther Immunol 1:3-15; and Honemann et al. (2004) Leukemia 18:636-644. Alternatively, the antibodies can be "linear antibodies" as described in, e.g., Zapata et al. (1995) Protein Eng. 8(10):1057-1062. Briefly, these antibodies comprise a pair of tandem Fd segments ($V_H$-$C_H$1-$V_H$-$C_H$1) that form a pair of antigen binding regions.

A bispecific antibody can also be a diabody. Diabody technology described by, e.g., Hollinger et al. (1993) Proc Natl Acad Sci USA 90:6444-6448 has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. *See also* Zhu et al. (1996) Biotechnology 14:192-196 and Helfrich et al. (1998) Int J Cancer 76:232-239. Bispecific single chain diabodies ("scDb") as well as methods for generating scDb are described in, e.g., Brüsselbach et al. (1999) Tumor Targeting 4:115-123; Kipriyanov et al. (1999) J Mol Biol 293:41-56; and Nettlebeck et al. (2001) Mol Ther 3:882-891.

Variant forms of bispecific antibodies such as the tetravalent dual variable domain immunoglobulin (DVD-Ig) molecules described in Wu et al. (2007) Nat Biotechnol 25(11):1290-1297 are also contemplated. The DVD-Ig molecules are designed such that two different light chain variable domains ($V_L$) from two different parent antibodies are linked in tandem directly or via a short linker by recombinant DNA techniques, followed by the light chain constant domain. Methods for generating DVD-Ig molecules from two parent antibodies are further described in, e.g., PCT Publication Nos. WO 08/024188 and WO 07/024715. Also embraced is the bispecific format described in, e.g., U.S. patent application publication no. 20070004909. Another bispecific format that can be used is the Cross-Over Dual V Region (CODV-Ig) which is a format for engineering four domain antibody-like molecules described in WO2012/135345. CODV-Ig was shown to be useful in engineering bispecific antibody-like molecules where steric hindrance at the C-terminal V domains (internal) may prevent construction of a DVD-Ig.

The eculizumab or an eculizumab variant inhibits complement component C5. In particular, they inhibit the generation of the C5a anaphylatoxin, or the generation of c5a and the C5b active fragments of a complement component C5 protein (e.g., a human C5 protein). Accordingly, they inhibit, e.g., the pro-inflammatory effects of C5a; and they inhibit the generation of the C5b-9 membrane attack complex ("MAC") at the surface of a cell and subsequent cell lysis. *See, e.g.,* Moongkarndi et al. (1982) Immunobiol 162:397 and Moongkarndi et al. (1983) Immunobiol 165:323.

Suitable methods for measuring inhibition of C5 cleavage are known in the art. For example, the concentration and/or physiologic activity of C5a and/or C5b in a body fluid can be measured by methods well known in the art. Methods for measuring C5a concentration or activity include, e.g., chemotaxis assays, RIAs, or ELISAs (see, e.g., Ward and Zvaifler (1971) J Clin Invest 50(3) :606-16 and Wurzner et al. (1991) Complement Inflamm 8:328-340). For C5b, hemolytic assays or assays for soluble C5b-9 known in the art can be used. Other assays known in the art can also be used.

Inhibition of complement component C5 can reduce the cell lysing ability of complement in a subject's body fluids. Such reductions of the cell-lysing ability of complement present can be measured by methods well known in the art such as, for example, by a conventional hemolytic assay such as the hemolysis assay described by Kabat and Mayer (eds), "Experimental Immunochemistry, 2nd Edition," 135-240, Springfield, IL, CC Thomas (1961), pages 135-139, or a conventional variation of that assay such as the chicken erythrocyte hemolysis method as described in, e.g., Hillmen et al. (2004) N Engl J Med 350(6):552.

Methods of making a recombinant CHO cells comprising an expression construct are known in the art. For example, nucleic acid encoding eculizumab or an eculizumab variant can be inserted into an expression vector that contains transcriptional and translational regulatory sequences, which include, e.g., promoter sequences, ribosomal binding sites, transcriptional start and stop sequences, translational start and stop sequences, transcription terminator signals, polyadenylation signals, and enhancer or activator sequences. The regulatory sequences include a promoter and transcriptional start and stop sequences. In addition, the expression vector can include more than one replication system such that it can be maintained in two different organisms, for example in mammalian or insect cells for expression and in a prokaryotic host for cloning and amplification. The regulatory sequences can allow either inducible or constitutive expression of the recombinant protein in a CHO cell. The expression construct is then inserted into a CHO cell, by methods well known in the art, such as transfection and electroporation.

Several possible vector systems (such as plasmid vector systems) well known in the art are available for the expression of eculizumab or an eculizumab variant from nucleic acids in CHO cells.

The expression vectors can be introduced by methods well known in the art into CHO cells in a manner suitable for subsequent expression of the nucleic acid. In some embodiments, the vectors and/or cells can be configured for constitutive, inducible, or repressible expression of eculizumab or an eculizumab variants by methods known in the art (see, e.g., Schockett et al. (1996) Proc Natl Acad Sci USA 93: 5173-5176 and U.S. Patent No. 7,056,897).

The eculizumab or an eculizumab variant can be produced from CHO cells by culturing a host CHO cell transformed with the expression vector containing nucleic acid encoding the polypeptides, under conditions, and for an amount of time, sufficient to allow expression of the proteins. Such conditions for protein expression, including constitutive or inducible expression, will vary with the choice of the expression vector and the host cell, and will be easily ascertained by one skilled in the art through routine experimentation. *See, e.g.,* Current Protocols in Molecular Biology, Wiley & Sons, and Molecular Cloning--A Laboratory Manual --3rd Ed., Cold Spring Harbor Laboratory Press, New York (2001), which has comprehensive disclosure of recombinant DNA technology.

Following expression, the eculizumab or an eculizumab variant can be isolated or purified in a variety of ways known to those skilled in the art.

Methods for determining whether an antibody binds, including "specifically binds," to an antigen and/or the affinity for an antibody to an antigen are known in the art. For example, the binding of an antibody to an antigen can be detected and/or quantified using a variety of techniques such as, but not limited to, Western blot, dot blot, surface plasmon resonance (SPR) method (e.g., BIAcore system; Pharmacia Biosensor AB, Uppsala, Sweden and Piscataway, N.J.), or enzyme-linked immunosorbent assay (ELISA). *See, e.g.,* Harlow and Lane (1988) "Antibodies: A Laboratory Manual" Cold Spring Harbor Laboratory Press, Cold Spring Harbor, N.Y.; Benny K. C. Lo (2004) "Antibody Engineering: Methods and Protocols," Humana Press (ISBN: 1588290921); Borrebaek (1992) "Antibody Engineering, A Practical Guide," W.H. Freeman and Co., NY; Borrebaek (1995) "Antibody Engineering," 2nd Edition, Oxford University Press, NY, Oxford; Johne et al. (1993) J Immunol Meth 160:191-198; Jonsson et al. (1993) Ann Biol Clin 51:19-26; and Jonsson et al. (1991) Biotechniques 11:620-627.

Methods of making, identifying, purifying, modifying, etc. eculizumab or an eculizumab variant are well known in the art. Methods of determining the glycan content and sialic acid content are also known in the art.

In certain embodiments, a typical therapeutic treatment includes a series of doses, which will usually be administered concurrently with the monitoring of clinical endpoints with the dosage levels adjusted as needed to achieve the desired clinical outcome. In certain embodiments, treatment is administered in multiple dosages over at least a few hours or a few days. In certain embodiments, treatment is administered in multiple dosages over at least a week. In certain embodiments, treatment is administered in multiple dosages over at least a month. In certain embodiments, treatment is administered in multiple dosages over at least a year. In certain embodiments, treatment is administered in multiple dosages over the remainder of the patient's life.

The frequency of administration can also be adjusted according to various parameters. These include, for example, the clinical response, the plasma half-life of the eculizumab or the eculizumab variant in a body fluid, such as, blood, plasma, serum, or synovial fluid. To guide adjustment of the frequency of administration, levels of the eculizumab or the eculizumab variant in the body fluid can be monitored during the course of treatment.

In certain embodiments, the dosage(s) and frequency of administration are determined according to the need of the patient, at the discretion of the treating physician.

In certain embodiments, a therapeutically effective amount of eculizumab or an eculizumab variant can include an amount (or various amounts in the case of multiple administrations) that improves the patient's chance of survival. In certain embodiments, a disclosed method improves the life expectancy of a patient by any amount of time, including at least one day, at least one week, at least two weeks, at least three weeks, at least one month, at least two months, at least three months, at least 6 months, at least one year, at least 18 months, at least two years, at least 30 months, or at least three years, or the duration of treatment.

In certain embodiments, a therapeutically effective amount of eculizumab or the eculizumab variant can include an amount (or various amounts in the case of multiple administrations) that improves symptoms of the condition or disease being treated. The eculizumab or the eculizumab variant is designed for treating or preventing a complement-associated disorder. Thus, the eculizumab or the eculizumab variant can be administered to a subject (e.g., a human) in need thereof in an amount sufficient to treat a complement-associated disorder afflicting the subject.

The complement-associated disorder can be, e.g., a complement-associated inflammatory disorder, paroxysmal nocturnal hemoglobinuria (PNH), atypical hemolytic uremic syndrome (aHUS), age-related macular degeneration (AMD), rheumatoid arthritis (RA), myasthenia gravis (MG), neuromyelitis optica (NMO), catastrophic anti-phospholipid syndrome (CAPS), anti-phospholipid syndrome (APS), viral hemorrhagic fever (such as Ebola hemorrhagic fever), or sepsis. In some embodiments, the complement-associated disorder is a complement-associated pulmonary disorder. For example, the complement-associated pulmonary disorder can be, e.g., asthma or chronic obstructive pulmonary disease (COPD). Other complement-associated disorders are also amenable to treatment or prevention.

Methods for determining whether eculizumab or an eculizumab variant inhibits C5 cleavage are known in the art. For inhibiting the formation of TCC, inhibition of human complement component C5 can reduce the cell-lysing ability of complement in a subject's body fluids. Such reductions of the cell-lysing ability of complement present in the body fluid(s) can be measured by methods well known in the art such as, for example, by a conventional hemolytic assay such as the hemolysis assay described by Kabat and Mayer (eds.), "Experimental Immunochemistry, 2nd Edition," 135-240, Springfield, IL, CC Thomas (1961), pages 135-139, or a conventional variation of that assay such as the chicken erythrocyte hemolysis method as described in, e.g., Hillmen et al. (2004) N Engl J Med 350(6): 552. Methods for determining whether a compound inhibits the cleavage of human C5 into forms C5a and C5b are known in the art and described in, e.g., Moongkarndi et al. (1982) Immunobiol 162:397; Moongkarndi et al. (1983) Immunobiol 165:323; Isenman et al. (1980) J Immunol 124(1):326-31; Thomas et al. (1996) Mol Immunol 33(17-18):1389-401; and Evans et al. (1995) Mol Immunol 32(16):1183-95. For example, the concentration and/or physiologic activity of C5a and C5b in a body fluid can be measured by methods well known in the art. Methods for measuring C5a concentration or activity include, e.g., chemotaxis assays, RIAs, or ELISAs (see, e.g., Ward and Zvaifler (1971) J Clin Invest 50(3):606-16 and Wurzner et al. (1991) Complement Inflamm 8:328-340). For C5b, hemolytic assays or assays for soluble C5b-9 known in the art can be used. Other assays known in the art can also be used.

Immunological techniques such as, but not limited to, ELISA can be used to measure the protein concentration of C5 and/or its cleavage products to determine the ability of a C5 inhibitor, such as an anti-C5 antibody, to inhibit conversion of C5 into biologically active products, for example, C5a generation.

Compositions containing eculizumab or an eculizumab variant can be formulated as a pharmaceutical composition for administering to a subject for treatment. Any suitable pharmaceutical compositions and formulations, as well as suitable methods for formulating and suitable routes and suitable sites of administration, are within the scope of this invention, and are known in the art. Also, any suitable dosage(s) and frequency of administration are contemplated.

The pharmaceutical compositions can include a pharmaceutically acceptable carrier. A "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, e.g., an acid addition salt or a base addition salt (see e.g., Berge et al. (1977) J Pharm Sci 66:1-19).

In certain embodiments, the protein compositions can be stabilized and formulated as a solution, microemulsion, dispersion, liposome, lyophilized (freeze-dried) powder, or other ordered structure suitable for stable storage at high concentration. Sterile injectable solutions can be prepared by incorporating a C5-binding polypeptide, for use in the methods of this invention, in the required amount in an appropriate solvent with one or a combination of ingredients enumerated above, as required, followed by filtered sterilization. Generally, dispersions are prepared by incorporating an eculizumab or an eculizumab variant into a sterile vehicle that contains a basic dispersion medium and the required other ingredients from those enumerated above. In the case of sterile powders for the preparation of sterile injectable solutions, methods for preparation include vacuum drying and freeze-drying that yield a powder of a C5 inhibitor polypeptide plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition a reagent that delays absorption, for example, monostearate salts, and gelatin. Non-protein C5 inhibitors can be formulated in the same, or similar, way.

The eculizumab or the eculizumab variant can be formulated at any desired concentration, including relatively high concentrations in aqueous pharmaceutical solutions. For example, eculizumab or of an eculizumab variant can be formulated in solution at a concentration of between about 10 mg/mL to about 100 mg/mL (e.g., between about 9 mg/mL and about 90 mg/mL; between about 9 mg/mL and about 50 mg/mL; between about 10 mg/mL and about 50 mg/mL; between about 15 mg/mL and about 50 mg/mL; between about 15 mg/mL and about 110 mg/mL; between about 15 mg/mL and about 100 mg/mL; between about 20 mg/mL and about 100 mg/mL; between about 20 mg/mL and about 80 mg/mL; between about 25 mg/mL and about 100 mg/mL; between about 25 mg/mL and about 85 mg/mL; between about 20 mg/mL and about 50 mg/mL; between about 25 mg/mL and about 50 mg/mL; between about 30 mg/mL and about 100 mg/mL; between about 30 mg/mL and about 50 mg/mL; between about 40 mg/mL and about 100 mg/mL; between about 50 mg/mL and about 100 mg/mL; or between about 20 mg/mL and about 50 mg/mL); or at any suitable concentration. The eculizumab or the eculizumab variant can be present in the solution at greater than (or at least equal to) about 5 (e.g., greater than, or at least equal to, about any of the following: 5, 6, 7, 8, 9, 10, 11, 12, 13, 14, 15, 16, 17, 18, 19, 20, 21, 22, 23, 24, 25, about 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, or even 150) mg/mL. Eculizumab or an eculizumab variant can be formulated at a concentration of greater than about 2 (e.g., greater than about any of the following: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 or more) mg/mL, but less than about 55 (e.g., less than about any of the following: 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, or less than about 5) mg/mL. Thus, in some embodiments, eculizumab or an eculizumab variant can be formulated in an aqueous solution at a concentration of greater than about 5 mg/mL and less than about 55 mg/mL. Eculizumab or an eculizumab variant can be formulated in an aqueous solution at a concentration of about 50 mg/mL. Any suitable concentration is contemplated. Methods for formulating a protein in an aqueous solution are known in the art and are described in, e.g., U.S. Patent No. 7,390,786; McNally and Hastedt (2007), "Protein Formulation and Delivery," Second Edition, Drugs and the Pharmaceutical Sciences, Volume 175, CRC Press; and Banga (1995), "Therapeutic peptides and proteins: formulation, processing, and delivery systems," CRC Press.

The dosage level for eculizumab or an eculizumab variant can be any suitable level. In certain embodiments, the dosage levels of eculizumab or an eculizumab variant, for human subjects can generally be between about 1 mg per kg and about 100 mg per kg per patient per treatment, and can be between about 5 mg per kg and about 50 mg per kg per patient per treatment.

The plasma concentration in a patient, whether the highest level achieved or a level that is maintained, of eculizumab or an eculizumab variant can be any desirable or suitable concentration. Such plasma concentration can be measured by methods known in the art. In certain embodiments, the concentration in the plasma of a patient (such as a human patient) of eculizumab or an eculizumab variant is in the range from about 25μg/mL to about 500 μg/mL (such as between, for example, about 35 μg/mL to about 100 μg/mL). Such a plasma concentration of an anti-C5 antibody, in a patient can be the highest attained after administering the anti-C5 antibody, or can be a concentration of an anti-C5 antibody in a patient that is maintained throughout the therapy. However, greater amounts (concentrations) may be required for extreme cases and smaller amounts may be sufficient for milder cases; and the amount can vary at different times during therapy. In certain embodiments, the plasma concentration of eculizumab or an eculizumab variant can be maintained at or above about 35 μg/mL during treatment. In some embodiments, the plasma concentration of the plasma concentration of eculizumab or an eculizumab variant can be maintained at or above about 50μg/mL during treatment.

In some embodiments, the plasma concentration of eculizumab or an eculizumab variant can be maintained at or above about 200nM, or at or above between about 280nM to 285nM, during treatment.

In other treatment scenarios, the plasma concentration of eculizumab or an eculizumab variant can be maintained at or above about 75μg/mL during treatment. In the most serious treatment scenarios, the plasma concentration of eculizumab or an eculizumab variant can be maintained can be maintained at or above about 100μg/mL during treatment.

In certain embodiments, the plasma concentration of eculizumab or an eculizumab variant can be maintained at or above about 200nM to about 430nM, or at or above about 570nM to about 580nM, during treatment.

In certain embodiments, the pharmaceutical composition is in a single unit dosage form. In certain embodiments, the single unit dosage form is between about 300 mg to about 1200 mg unit dosage form (such as about 300 mg, about 900 mg, and about 1200 mg) of eculizumab or an eculizumab variant. In certain embodiments, the pharmaceutical composition is lyophilized. In certain embodiments, the pharmaceutical composition is a sterile solution. In certain embodiments, the pharmaceutical composition is a preservative free formulation. In certain embodiments, the pharmaceutical composition comprises a 300 mg single-use formulation of 30 ml of a 10 mg/ml sterile, preservative free solution.

In certain embodiments, eculizumab or an eculizumab variant is administered according to the following protocol:

600 mg via 25 to 45 minute IV infusion every 7 +/- 2 days for the first 4 weeks, followed by 900 mg for the fifth dose 7±2 days later, then 900 mg every 14±2 days thereafter. The antibody can be administered via IV infusion over 25 to 45 minute. In another embodiment, eculizumab or an eculizumab variant is administered according to the following protocol: 900 mg via 25 to 45 minute IV infusion every 7 +/- 2 days for the first 4 weeks, followed by 1200 mg for the fifth dose 7±2 days later, then 1200 mg every 14±2 days thereafter. The antibody can be administered via IV infusion over 25 to 45 minute. An exemplary pediatric dosing of, for example, a full-length eculizumab antibody or a full-length eculizumab variant antibody, tied to body weight, is shown in Table 1:

Table 1 Exemplary dosing Recommendations in Pediatric Patients for Full-length Antibodies

| Patient Body Weight | Induction | Maintenance |
|---|---|---|
| 40 kg and over | 900 mg weekly X 4 doses | 1200 mg at week 5; then 1200 mg every 2 weeks |
| 30 kg to less than 40 kg | 600 mg weekly X 2 doses | 900 mg at week 3; then 900 mg every 2 weeks |
| 20 kg to less than 30 kg | 600 mg weekly X 2 doses | 600 mg at week 3; then 600 mg every 2 weeks |
| 10 kg to less than 20 kg | 600 mg weekly X 1 dose | 300 mg at week 2; then 300 mg every 2 weeks |
| 20 kg to less than 30 kg | 600 mg weekly X 1 dose | 600 mg at week 2; then 600 mg every 3 weeks |

Note that in certain other embodiments the eculizumab or eculizumab variant that is not a full-length antibody and is smaller than a full-length antibody can be administered at a dosage that correspond to the same molarity as the dosage for a full-length antibody.

The aqueous solution can have a neutral pH, e.g., a pH between, e.g., about 6.5 and about 8 (e.g., between and inclusive of 7 and 8). The aqueous solution can have a pH of about any of the following: 6.6, 6.7, 6.8, 6.9, 7, 7.1, 7.2, 7.3, 7.4, 7.5, 7.6, 7.7, 7.8, 7.9, or 8.0. In some embodiments, the aqueous solution has a pH of greater than (or equal to) about 6 (e.g., greater than or equal to about any of the following: 6.1, 6.2, 6.3, 6.4, 6.5, 6.6, 6.7, 6.8, 6.9, 7, 7.1, 7.2, 7.3, 7.4, 7.5, 7.6, 7.7, 7.8, or 7.9), but less than about pH 8.

In some embodiments, the eculizumab or the eculizumab variant is administered intravenously to the subject (the term "subject" is used herein interchangeably with the term "patient"), including by intravenous injection or by intravenous infusion. In some embodiments, the eculizumab or the eculizumab variant is administered intravenously to the subject, including by intravenous infusion. In some embodiments, the eculizumab or the eculizumab variant is administered to the lungs of the subject. In some embodiments, the eculizumab or the eculizumab variant is administered to the subject by subcutaneous injection. In some embodiments, the eculizumab or the eculizumab variant is administered to the subject by way of intraarticular injection. In some embodiments, the eculizumab or the eculizumab variant is administered to the subject by way of intravitreal or intraocular injection. In some embodiments, the eculizumab or the eculizumab variant is administered to the subject by pulmonary delivery, such as by intrapulmonary injection (especially for pulmonary sepsis). Additional suitable routes of administration are also contemplated.

Eculizumab or an eculizumab variant can be administered to a subject as a monotherapy. In some embodiments, the methods described herein can include administering to the subject one or more additional treatment, such as one or more additional therapeutic agents.

The additional treatment can be any additional treatment, including experimental treatment for a complement-associated disorder, or a treatment for a symptom of a complement-associated disorder. The other treatment can be any treatment, any therapeutic agent, which improves or stabilizes the patient's health. The additional therapeutic agent(s) includes IV fluids, such as water and/or saline, acetaminophen, heparin, one or more clotting factors, antibiotics, etc. The one or more additional therapeutic agents can be administered together with the eculizumab or the eculizumab variant or as separate therapeutic compositions or one therapeutic composition can be formulated to include both: (i) one or more eculizumab and eculizumab variant and (ii) one or more additional therapeutic agents. An additional therapeutic agent can be administered prior to, concurrently, or after administration of the C5-binding polypeptide. An additional agent can be administered using the same delivery method or route or using a different delivery method or route. The additional therapeutic agent can be another complement inhibitor, including another C5 inhibitor.

When eculizumab or an eculizumab variant is to be used in combination with a second active agent, the agents can be formulated separately or together. For example, the respective pharmaceutical compositions can be mixed, e.g., just prior to administration, and administered together or can be administered separately, e.g., at the same or different times, by the same route or different route.

In some embodiments, a composition can be formulated to include a sub-therapeutic amount of eculizumab or an eculizumab variant and a sub-therapeutic amount of one or more additional active agents such that the components in total are therapeutically effective for treating a complement-associated disorder. Methods for determining a therapeutically effective dose of an agent such as a therapeutic antibody are known in the art.

The compositions can be administered to a subject, e.g., a human subject, using a variety of methods that depend, in part, on the route of administration. The route can be, e.g., intravenous ("IV") injection or infusion, subcutaneous ("SC") injection, intraperitoneal ("IP") injection, pulmonary delivery such as by intrapulmonary injection (especially for pulmonary sepsis), intraocular injection, intraarticular injection, or intramuscular ("IM") injection.

A suitable dose of eculizumab or an eculizumab variant disclosed herein can depend on a variety of factors including, e.g., the age, gender, and weight of a subject to be treated and the particular inhibitor compound used. Other factors affecting the dose administered to the subject include, e.g., the type or severity of the illness. Other factors can include, e.g., other medical disorders concurrently or previously affecting the subject, the general health of the subject, the genetic disposition of the subject, diet, time of administration, rate of excretion, drug combination, and any other additional therapeutics that are administered to the subject. It should also be understood that a specific dosage and treatment regimen for any particular subject will depend upon the judgment of the treating medical practitioner (e.g., doctor or nurse).

Eculizumab or an eculizumab variant, disclosed herein, can be administered as a fixed dose, or in a milligram per kilogram (mg/kg) dose. In some embodiments, the dose can also be chosen to reduce or avoid production of antibodies or other host immune responses against one or more of the active antibodies in the composition.

A pharmaceutical composition can include a therapeutically effective amount of eculizumab or an eculizumab variant disclosed herein. Such effective amounts can be readily determined by one of ordinary skill in the art.

In certain embodiments, the dosing of eculizumab or a variant thereof, disclosed herein, can be as follows: (1) administering to patient about 900 milligrams (mg) of eculizumab or a variant thereof each week for the first 3 weeks, or (2) 1200 milligrams (mg) of eculizumab or a variant thereof each week for the first 3 weeks and (3) followed by an about 1200 mg dose on weeks 4, 6, and 8. After an initial 8-week eculizumab or a variant thereof treatment period, the treating medical practitioner (such as a physician) can optionally request (and administer) treatment with eculizumab or a variant thereof about 1200 mg every other week for an additional 8 weeks. The patient can then be observed for 28 weeks following eculizumab or a variant thereof treatment.

While in no way intended to be limiting, exemplary methods of administration for a single chain antibody such as a single chain anti-C5 antibody (that inhibits cleavage of C5) are described in, e.g., Granger et al. (2003) Circulation 108:1184; Haverich et al. (2006) Ann Thorac Surg 82:486-492; and Testa et al. (2008) J Thorac Cardiovasc Surg 136(4):884-893.

The terms "therapeutically effective amount" or "therapeutically effective dose," or similar terms used herein are intended to mean an amount of eculizumab or an eculizumab variant disclosed herein that will elicit the desired biological or medical response.

In some embodiments, a composition described herein contains a therapeutically effective amount of eculizumab or an eculizumab variant disclosed herein, and one or more (e.g., one, two, three, four, five, six, seven, eight, nine, ten, or eleven or more) additional therapeutic agents such that the composition as a whole is therapeutically effective. For example, a composition can contain a C5-binding polypeptide described herein and an immunosuppressive agent, wherein the polypeptide and agent are each at a concentration that when combined are therapeutically effective for treating or preventing a complement-associated disorder in a subject.

In this disclosure, reference to eculizumab or an eculizumab variant in methods disclosed herein, and treatment and prevention using the eculizumab or the eculizumab variant, are references to the eculizumab disclosed herein and to the eculizumab variant disclosed herein.

A "subject," as used herein, can be a human. A "patient" is used herein interchangeably with a "subject." In certain embodiments, the patient (or the subject) is a human patient (or human subject).

**Examples**

For this invention to be better understood, the following examples are set forth. These examples are for purposes of illustration only and are not to be construed as limiting the scope of the invention in any manner.

**Example 1. Eculizumab Treatment**

From 1 mg per kg to 100 mg per kg per patient per treatment of a formulation comprising eculizumab disclosed herein are administered to human patients diagnosed with a complement disorder by intravenous infusion. All of these patients are administered eculizumab for the first time early on in the disease state. At various days after, the disease level is determined by any methods known in the art.

The life expectancy of the patients receiving the formulation comprising eculizumab disclosed herein is increased by at least one day.

**Example 2. Clinical Trial**

A clinical trial enrolls 100 patients with a complement disorder. Patients in the study receive 1200 milligrams (mg) of an eculizumab disclosed herein on day 1 of the study, followed by 1200 mg each week for the next 2 weeks, followed by a 1200 mg dose on weeks 4, 6, and 8. After an initial 8-week eculizumab treatment period, study investigators can optionally request treatment with eculizumab 1200 mg disclosed herein every other week for an additional 8 weeks. The administration to patients of the eculizumab disclosed herein is performed by intravenous infusion.

The life expectancy of the patients receiving eculizumab disclosed herein is increased by at least one day.

**Example 3. A Procedure for Determining the Monosaccharide Composition of Eculizumab or an Eculizumab Variant in aqueous solution**

**Equipment and Materials**

Reagents: Glucosamine hydrochloride (Sigma-Aldrich, G4875); Galactosamine hydrochloride (Sigma-Aldrich, G0500) Glucose (Sigma-Aldrich, G7528); Galactose (Sigma-Aldrich, G6404) Mannose (Sigma-Aldrich, M6020); Fucose (Sigma-Aldrich, F2252); Acetic acid, glacial (Sigma-Aldrich, 320099); Sodium acetate (Sigma-Aldrich, S7545); Boric acid (Sigma-Aldrich, B7660); Methanol (Sigma-Aldrich, 439193); Anthranilic acid (Sigma-Aldrich, A89855); Sodium cyanoborohydride (Sigma-Aldrich, 156159); Butylamine (Sigma-Aldrich, 471305); Phosphoric acid (Sigma-Aldrich, 695017); Tetrahydrofuran (THF) (Sigma-Aldrich, 494461); Deionized water (diH$_2$O); Acetonitrile, HPLC grade (Sigma-Aldrich, 360457).

HPLC Mobile Phase: Mobile Phase A: 0.2% butylamine, 0.5% phosphoric acid and 1% THF in diH$_2$O; Mobile Phase B: 50% Mobile Phase A in 50% acetonitrile; Mobile Phase C: 40% methanol in diH$_2$O.

Equipment: Waters Alliance 2695 HPLC system, equipped with temperature control module (TCM) or column oven and model 2475 multi-wavelength fluorescence detector; Waters Empower Chromatography Software; Waters Symmetry C-18 column, 150 x 4.6mm, 5$\mu$m particle size (WAT045905); Vivaspin 500 filters, 10kDa MWCO PES, Sartorius-Stedim # VS0102; Eppendorf micro-centrifuge 2.0mL microcentrifuge tubes with O-ring seal screw-caps, BioStor, National Scientific; 2-1000pL pipettes, Rainin; Vacuum-concentrator centrifuge; Dry heating block; Screw neck injection vials with caps, Waters.

**Procedures**

Preparation of Solvents and Reagents.

1.0mM Monosaccharide Standard Stock Solution: Weigh 43 mg each of glucosamine hydrochloride and galactosamine hydrochloride. Weigh 36 mg each of galactose, mannose and glucose. Weigh 33 mg of fucose. Combine monosaccharides and add diH$_2$O to final volume of 200mL. Mix until dissolved and aliquot into 1mL vials. Store at -20°C. Expires in 5 years.

Monosaccharide Standard Calibration Solution (0.04mM): Thaw an aliquot of 1.0mM monosaccharide standard stock solution to room temperature. Add 40pL of 1.0mM standard stock to 960pL of diH$_2$O and mix well. Prepare fresh.

0.05 % Acetic Acid: Add 50 $\mu$L of glacial acetic acid to 100 mL of diH$_2$O. Store at room temperature. Expires in 1 year.

1% Sodium Acetate: Dissolve 1g of sodium acetate in 100mL of diH$_2$O. Store at room temperature. Expires in 1 year.

4% Sodium Acetate, 2% Boric Acid, Methanolic Solution: Dissolve 4g of sodium acetate and 2g of boric acid in 100mL of methanol. Store at room temperature. Expires in 1 year.

30 mg/mL Anthranilic Acid and 20 mg/mL Sodium Cyanoborohydride Solution (AA Solution): Dissolve 200mg of sodium cyanoborohydride in 10mL sodium acetate/ boric acid/ methanolic solution (see 6.2.5, above) followed by the addition of 300mg of anthranilic acid. Mix well. Prepare fresh.

HPLC Mobile Phase A: Mix 4 mL of butylamine, 10 mL of phosphoric acid and 20 mL of THF into 1.8L of diH$_2$O. QS to 2L with H$_2$O and mix well. Store at room temperature. Expires in 6 months.

HPLC Mobile Phase B: Add 500 mL of Mobile Phase A to 500 mL of acetonitrile. Mix well. Store at room temperature. Expires in 6 months.

HPLC Mobile Phase C: Add 400mL of methanol to 600mL of H$_2$O. Mix well. Store at room temperature. Expires in 6 months.

Sample Hydrolysis: Add 500$\mu$L of water to a spin filter. Centrifuge at 12000rpm for 5min to rinse. Discard filtrate. Place 4mg of sample (400$\mu$L at 10mg/mL) into the rinsed filter. QS to 500$\mu$L with 0.05 % AcOH solution. Centrifuge at 12000rpm for 8-10 min. Discard filtrate, QS to 500μL with 0.05% AcOH solution and centrifuge at 12000rpm for 8-10 min. Repeat for a total of two wash steps. Adjust the final volume to approximately 250μL with 0.05% AcOH solution. Vortex and transfer to microfuge tube. Pool replicates as needed. Make a 1:10 dilution for UV concentration determination by adding 50μL of the protein to 450μL of 0.05% AcOH solution. Blank the UV detector with 0.05% AcOH solution. Measure absorbance at 280nm of each 1:10 diluted protein sample. Determine the protein concentration in mg/mL by multiplying the A280 by the dilution factor of 10 and by the absorbance factor specific to the protein with regard to cell path length. Samples may be stored at 4°C before continuing hydrolysis.

Dilute the protein solution in 0.05% AcOH to a final concentration of approximately 0.5mg/mL. In BioStor tubes mix 0.1 mL of the 0.5mg/mL protein solution, 0.3 mL of $diH_2O$ and 0.1 mL of TFA. Cap tubes tightly, mix well and incubate at 100°C for 6 hours. After tubes cool to room temperature, dry contents in a vacuum-concentrator to dryness without heat. Samples may be stored at 4°C prior to tagging.

Standard Curve Preparation: Prepare the standard curve in tubes by the dilution series, Table 2, using the 0.04mM Monosaccharide Standard Calibration Solution.

**Table 2: Standard Curve Dilution Series**

| Target Conc. (mM) | 0.040 | 0.030 | 0.020 | 0.010 | 0.001 |
|---|---|---|---|---|---|
| Vol. of $diH_2O$ (μL) | | 103 | 103 | 110 | 180 |
| Vol. of Previous Sol'n (μL) | | 308 | 207 | 110 | 20 |
| **On-column Standard Level (pmol)** | **100** | **75** | **50** | **25** | **2.5** |

Sample Tagging: Dissolve dried samples in 100pL of 1% sodium acetate by vortexing and sonication. Place 50μL each of Monosaccharide Standard Dilutions into separate screw-cap plastic vials. Split volume of hydrolyzed protein from Step 6.5.1 into two 50μL aliquots and place into separate screw-cap plastic vials. Add 100pL of AA solution from above, to each vial and incubate at 80°C in heating blocks for 1 hour. After the reaction, allow contents to cool to room temperature and add 850pL of HPLC Solvent A. Mix well and transfer contents to autosampler vials.

Running Samples: Equilibrate column with 5% B at 1.0 mL/min for 30 min. Running conditions are as follows: TCM or column oven:20 ± 5°C; Sample Tray Temperature: 5 ± 2°C; Degasser Unit: Normal; Sampler Interval: 1.0 sec; Wavelength: 360nm excitation, 420nm emission; Flow: 1.0 mL/min; Run Time: 85 min. Set processing to "RUN ONLY".

**Table 3, Gradient:**

| Time (min) | % B | Curve |
|---|---|---|
| 0 .0 | 5 | 6 |
| 22.0 | 5 | 6 |
| 50.0 | 15 | 6 |
| 50.1 | 100 | 6 |
| 70.0 | 100 | 6 |
| 70.1 | 5 | 6 |
| 85.0 | 5 | 6 |

Inject 50 μL each of Solvent A blank, standard curve and test samples:

**Table 4**

| Inj Vol (μL) | Sample Name | Sample Type | Level | Function | Method Set |
|---|---|---|---|---|---|
| 50.0 | blank- Solvent A | Unknown | | Inject Samples | Mono |
| 50.0 | Mono Std 2.5pmol | Standard | 1 | Inject Standards | Mono |
| 50.0 | Mono Std 25pmol | Standard | 2 | Inject Standards | Mono |
| 50.0 | Mono Std 50pmol | Standard | 3 | Inject Standards | Mono |

(continued)

| Inj Vol (μL) | Sample Name | Sample Type | Level | Function | Method Set |
|---|---|---|---|---|---|
| 50.0 | Mono Std 75pmol | Standard | 4 | Inject Standards | Mono |
| 50.0 | Mono Std 100pmol | Standard | 5 | Inject Standards | Mono |
| 50.0 | reference replicate #1 | Unknown | | Inject Samples | Mono |
| 50.0 | reference replicate #2 | Unknown | | Inject Samples | Mono |
| 50.0 | sample replicate #1 | Unknown | | Inject Samples | Mono |
| 50.0 | sample replicate #2 | Unknown | | Inject Samples | Mono |

Wash column with Solvent C for 15 min and store it in 100 % Solvent B prior to shutdown of the system.

Data Processing and Analysis: Clear previous calibration curves if needed, calibrate the standard curve and then process samples. Processing method amounts for standard levels are as follows:

**Table 5**

| Level | Glucosamine | Galactosamine | Galactose | Mannose | Glucose | Fucose |
|---|---|---|---|---|---|---|
| 1 | 2.5 | 2.5 | 2.5 | 2.5 | 2.5 | 2.5 |
| 2 | 25 | 25 | 25 | 25 | 25 | 25 |
| 3 | 50 | 50 | 50 | 50 | 50 | 50 |
| 4 | 75 | 75 | 75 | 75 | 75 | 75 |
| 5 | 100 | 100 | 100 | 100 | 100 | 100 |

Processing parameters are as follows: Threshold: 20 μv/sec; Peak Width: 150 sec; Inhibit Integration: 0.0 - 6.0 min, 10 - 22 min. and 40.0 - 85.0 min.

Label peaks as follows:

**Table 6**

| Peak | Approximate Retention Time (min.) |
|---|---|
| Glucosamine | 7-8 |
| Galactosamine | 8-9 |
| Galactose | 24-25 |
| Mannose | 27-28 |
| Glucose | 29-30 |
| Fucose | 37-38 |

Retention times may vary and processing parameters may be adjusted, if necessary.

**Calculations:** Weighted Theoretical Molar Ratio Calculation. Using the results from the oligosaccharide assay of the reference material, calculate the average of relative percent area of each oligosaccharide. The weighted theoretical molar ratio for each monosaccharide type is the sum of the products of each averaged oligosaccharide percent area multiplied by the number of molecules of monosaccharide per oligosaccharide type, then divided by 100. The weighted theoretical molar ratio normalized to three mannose molecules for each monosaccharide is the product of the weighted theoretical molar ratio of the monosaccharide multiplied by three, then divided by the weighted theoretical molar ratio of mannose. See **Table 7** for example.

**Table 7:** Weighted Theoretical Molar Ratio Calculation

| Oligosaccharid e | Avg. Relative % Area from Oligo Assay | # Molecules of Mono per Oligo | | | | |
|---|---|---|---|---|---|---|
| | | **GlcNAc** | **GalNac** | **Galactose** | **Mannose** | **Fucose** |
| **M3N2F** | 0.00 | 2.00 | 0.00 | 0.00 | 3.00 | 1.00 |
| **G0-GN/G0F-GN** | 4.99 | 3.00 | 0.00 | 0.00 | 3.00 | 1.00 |
| **G0/G0F** | 67.24 | 4.00 | 0.00 | 0.00 | 3.00 | 1.00 |
| **G1/G1F** | 17.38 | 4.00 | 0.00 | 1.00 | 3.00 | 1.00 |
| **G2/G2F/Man5** | 6.21 | 3.33 | 0.00 | 2.00 | 3.60 | 1.00 |
| **M6** | 0.11 | 2.00 | 0.00 | 0.00 | 6.00 | 0.00 |
| **M7** | 0.02 | 2.00 | 0.00 | 0.00 | 7.00 | 0.00 |
| **M8** | 0.03 | 2.00 | 0.00 | 0.00 | 8.00 | 0.00 |
| **M9** | 0.00 | 2.00 | 0.00 | 0.00 | 9.00 | 0.00 |
| **1SA** | 3.42 | 4.00 | 0.00 | 2.00 | 3.00 | 1.00 |
| **2SA** | 0.08 | 4.00 | 0.00 | 2.00 | 3.00 | 1.00 |
| **aGal** | 2.73 | 4.00 | 0.00 | 2.00 | 3.00 | 1.00 |
| **Total \*** | 102.20 | | | | | |
| **Weighted Theoretical Molar Ratio** | | 3.99 | 0.00 | 0.42 | 3.11 | 0.99 |
| **Weight. Theor. Molar Ratio (normalized to 3 mannose)** | | 3.85 | 0.00 | 0.41 | 3.00 | 0.96 |
| Note: *Totals may be over 100% as SA1/aGal4 is counted as both SA and aGal. | | | | | | |

Monosaccharide Calculations: Calculate test sample monosaccharide amounts in pmols based on each calibrated standard curve. Calculate the average peak area for duplicate samples. Report the ratio of monosaccharide (in nmol) per protein (in mg). See **Table 8** for example. Note that the Calculations may be adjusted as needed and documented in the laboratory notebook. Glucose is not included in the final % glycosylation total.

**Table 8: Monosaccharide Calculations**

| Sample | Name | Amount #1 (pmol) | Amount #2 (pmol) | Average Amount (pmol) | nmoles Mono to mg Protein (nmol/mg) | Amt. Mono to Amt. Protein (mol/mol) | Experimental Ratio (normalized to moles of mannose) | Weighted Theoretical Molar Ratio (normalized to moles of mannose) |
|---|---|---|---|---|---|---|---|---|
| | GlcN | 45.05 | 45.03 | 45.04 | 36.69 | 5.42 | 4.66 | 3.85 |
| | GalN | 0.00 | 0.00 | 0.00 | 0.00 | 0.00 | 0.00 | 0.00 |
| | Gal | 3.16 | 3.65 | 3.41 | 2.78 | 0.41 | 0.35 | 0.41 |
| RS5000xxxx | Man | 29.00 | 29.04 | 29.02 | 23.64 | 3.50 | 3.00 | 3.00 |
| | Glu | 5.31 | 5.27 | nc | nc | nc | nc | 0.00 |
| | Fuc | 9.98 | 9.99 | 9.98 | 8.13 | 1.20 | 1.03 | 0.96 |
| | Total | 92.49 | 92.99 | 87.45 | 71.23 | 10.53 | 9.04 | 8.22 |

Total percent glycosylation:

$$\% \text{ Glycosylation (w:w)} = \frac{\Sigma \text{ (g mono/ mol protein)}}{\text{MW protein}} \times 100$$

See **Table 9** for example.

**Table 9:** Total Percent Glycosylation:

| Name | MW of sugar (Da) | Mono to Protein (g/mol) |
|---|---|---|
| Glucosamine | 211 | 1144.61 |
| Galactose | 180 | 73.87 |
| Mannose | 180 | 629.12 |
| Fucose | 164 | 197.20 |
| Total | | 2044.81 |
| % Glycosylation (w:w) | | 1.38 |

Calculations may be adjusted as needed and documented in the laboratory notebook. Glucose is not included in the final % glycosylation total.

Equivalent equipment and materials may be used. And the procedures may be adjusted as needed.

**Example 4: A Procedure for N-linked Oligosaccharide Profiling using 2-AA Labeling by HPLC.**

The procedure applies to the characterization of protein, such as eculizumab or an eculizumab variant, in aqueous solution.

**Equipment and Materials**

Equipment: Waters Alliance 2695/2795 HPLC system equipped with a model 2475 multi-wavelength fluorescence detector; Waters Empower Chromatography Software; Showa Denko Asahipak Amino Column, 250 x 4.6mm, 5μm particle size, NH2P-504E (Phenomenex CHO-2628); Centrifuge, Eppendorf model 5415C or equivalent; Dry heating block (37°C, 80°C and 100°C); Vortex mixer; Polypropylene rack (VWR 60985-545).

Materials: Reference Standard or Control; Deionized water (diH$_2$O); Acetonitrile, HPLC grade (Burdick & Jackson 014-4); Peptide N-Glycosidase F (Prozyme, N-Glycanase, GKE-5006B or GKE-5006D; Ammonium Hydroxide (Baker 9721-02); Anthranilic Acid (Aldrich A8985-5); Acetic Acid, glacial (Baker 9526-03); Boric Acid (Aldrich 20287-8); Man9 2-AA (100pmol) (Prozyme GKSA-107); Sodium Acetate (Fluka 71179); Sodium Cyanoborohydride (Sigma 156159); β-mercaptoethanol (Bio-Rad 161-0710); Methanol, HPLC grade (Baker 9070-3); Ethanol, HPLC grade (Spectrum E1028); Igepal CA-630 (Sigma I-3021); Sodium Dodecyl Sulfate, SDS (Baker 4095-02); Tetrahydrofuran, THF, inhibited (Sigma 360589); Triethylamine, TEA (Sigma 471283); 2.0mL microcentrifuge tubes with O-ring seal screw-caps (Biostor, National Scientific, VWR 66006-812); 1.5mL microcentrifuge tubes; 0.45pm, 25mm Nylon Acrodisc Filters (Pall PN AP-4438T); Total recovery glass HPLC vials (Waters 186000384c); 5mL syringe (Becton-Dickinson 309603) .

**Procedures**

Preparation of Solvents, Reagents and Mobile Phases

1:100 Ammonium Hydroxide Solution: Add 50μL of ammonium hydroxide to 4.95mL of water and mix well. Store at 2-8°C. Expires in 1 month.

10% SDS Solution (prepare in a chemical fume hood): Add 10g of SDS to 80mL of water. QS to 100mL with water and mix well. Store at room temperature. Expires in 1 year.

Denaturing Solution: Add 4.75mL of 1:100 ammonium hydroxide solution to a 15mL polypropylene tube. Add 250pL of 10% SDS. Add 50μL of β-mercaptoethanol and mix well. Store at 2-8°C. Expires in 1 month.

5% Igepal: Add 5g of Igepal to 80mL of water. QS to 100mL with water and mix well. Store at room temperature. Expires in 1 year.

Methanolic Solution: Add 4g of sodium acetate and 2g of boric acid to 90mL of methanol. QS to 100mL with methanol and mix well. Store at room temperature. Expires in 1 year.

AA Solution (prepare fresh): Place 10mL of methanolic solution in a 15mL polypropylene tube. Add 200mg of sodium cyanoborohydride to the solution and mix until dissolved. Add 300mg of anthranilic acid to the solution and mix until dissolved. Store at 2-8°C. Discard if precipitate forms before use.

Wash Solvent: Add 5mL of water to 95mL acetonitrile and mix well. Store at room temperature. Expires in 6 months.

Elution Solvent: Add 20mL of acetonitrile to 80mL of water. Mix well. Store at room temperature. Expires in 6 months.

Mobile Phase A: Add 40mL of acetic acid and 20mL THF to 1600mL acetonitrile. QS to 2L with acetonitrile and mix well. Store at room temperature. Expires in 6 months.

Mobile Phase B: Add 100mL acetic acid, 20mL THF and 60mL TEA to 1600mL water. QS to 2L with water and mix well. Store at room temperature. Expires in 6 months.

Column Wash: Add 400mL ethanol to 1500mL water. QS to 2L with water and mix well. Store at room temperature. Expires in 6 months.

Sample, Reference and Negative Control Preparation: Pipette 25pg of each sample and reference into 2mL screw-cap tubes. (Initial sample amount may be increased for samples showing a lower intensity profile or for those with low levels of glycosylation.) Use 5pL of water for negative control. Add 30pL of denaturing solution to each tube and vortex. Incubate at 100°C for 2 minutes. **Caution: Do not open hot tubes.** Cool at room temperature for 5 minutes. Add 10pL of Igepal solution to each tube and vortex. Add 2pL of N-Glycanase enzyme, vortex and centrifuge to bring to bottom of tube. Incubate at 37°C for 16-22 Prepare fresh AA solution Add 100pL of AA solution to each tube and vortex. Incubate at 80°C for 1hr. For each sample, attach an Acrodisc filter to a 5mL syringe and place on a polypropylene rack. Discard syringe plungers. Cool reaction mixtures at room temperature for 5 minutes. Apply 1mL of Wash solvent to each filter. Do not allow filters to dry out during the gravity-driven extraction. Add 1mL of Wash solvent to each sample and vortex. Apply entire volume to filters and drain. Apply 2mL of Wash solvent to filters and drain. Elute with 1mL of Elution solvent directly into 1.5mL microfuge tubes. Transfer samples, reference and negative control to HPLC vials. Store remaining samples, reference and negative control at -20°C or below.

System Suitability Preparation: Add 100pL of water to tube containing 100pmol Man-9 AA and vortex. Solution concentration is 1pmol/$\mu$L. Store at -20°C or below. Expires in 1 year. Add 40pL of Man-9 solution to 360pL of Elution solution and vortex. Transfer to an HPLC vial.

HPLC System Setup: Equilibrate column with 70% A / 30% B at 1.0mL/min for 30 min. Set the following conditions: TCM or column oven: 50°C $\pm$ 5°C; Sample tray temperature: 5°C $\pm$ 2°C; Degasser Unit: normal; Sampling Interval: 1.0 sec. Wavelength: 360nm excitation, 420nm emission; Gain: 1; Time Constant: 0.3; Detector Output Units: Emmisions; Run Time: 110min.

Gradient:

**Table 10**

| Time (min.) | Flow rate (mL/min.) | % A | % B | Curve |
|---|---|---|---|---|
| Initial | 1.0 | 70 | 30 | 6 |
| 2 | 1.0 | 70 | 30 | 6 |
| 62 | 1.0 | 20 | 80 | 6 |
| 63 | 1.0 | 5 | 95 | 6 |
| 78 | 1.0 | 5 | 95 | 6 |
| 78.1 | 1.0 | 70 | 30 | 6 |
| 112 | 1.0 | 70 | 30 | 6 |
| 113 | 0 | 70 | 30 | 11 |

Arrange Sample Set as follows: **Table 11**

| Vial Number | Sample Name | Number of Injections | Injection Volume |
|---|---|---|---|
| 1 | Blank- water or Mobile Phase A | 1 | 100 |
| 2 | Negative Control | 1 | 100 |
| 3 | Man9-AA (Sys Suit) | 1 | 100 |
| 4 | Reference | 1 | 100 |
| 5 | Test Samples | 1 | 100 |

Running Samples: Multiply system suitability samples may be added. Set processing to "Run Only". At the end of the run, wash column with Column Wash for 15 minutes prior to shutdown of the system.

Processing:

Set processing parameters as follows: Enable Apex Track: Start: 16, End: 55. Minimum Area: 5000$\mu$V* sec; Minimum Height: 500$\mu$V; Liftoff: 0.5; Touchdown: 0.5; Threshold: 100$\mu$V/sec; Peak Width: 20sec; Inhibit Integration: 0.0 to 19.5min and > 60min.

Label peaks as follows:

**Table 12**

| Peak Name | Estimated Retention Time (min.) | Group Name |
|---|---|---|
| G0F-GN | 20.4 | Neutral/ Asialo |
| G0F | 22.2 | Neutral/ Asialo |
| G1F | 23.6 | Neutral/ Asialo |
| G2F* | 24.7 | Neutral/ Asialo |
| Man5 | 25.2 | Neutral/ Asialo |
| aGal1 | 26.3 | Neutral/ Asialo |
| Man6 | 26.7 | Neutral/ Asialo |
| aGal2 | 27.6 | Neutral/ Asialo |
| Man7 | 28.0 | Neutral/ Asialo |
| aGal3 | 29.0 | Neutral/ Asialo |
| Man8 | 30.0 | Neutral/ Asialo |
| Man9 | 31.0 | Neutral/ Asialo |
| SA1-1 | 38.3 | Mono-sialylated (acidic) |
| SA1/aGal4 | 38.8 | Mono-sialylated (acidic) |
| SA1-2 | 39.8 | Mono-sialylated (acidic) |
| SA1-3 | 41.0 | Mono-sialylated (acidic) |
| SA1-4 | 42.2 | Mono-sialylated (acidic) |
| SA2-1 | 54.0 | Di-sialylated |
| SA2-2 | 54.3 | Di-sialylated |
| *G2F is dependent on host cell type and may not be present in all samples. | | |

Some manual integration may be necessary. Retention times may be adjusted. Some peaks may not be detected in all samples.

Calculate the relative percent area of each peak as needed.

Equivalent equipment and materials may be used. And the procedures may be adjusted as needed.

**Example 5: A Procedure for the Determination of Sialic Acid Content of Eculizumab or an Eculizumab variant in Aqueous Solution.**

**Equipment and Materials**

Reagents: Acetic acid, glacial (Sigma-Aldrich, 320099); Deionized water (diH$_2$O); Sodium bisulfate (Sigma-Aldrich, 71657); N-acetylneuraminic acid, NANA (Sigma-Aldrich, A2388); N-glycolneuraminic acid, NGNA (Sigma-Aldrich, G9793); O-phenylenediamine 2HCl (Sigma-Aldrich, P1526); Butylamine (Sigma-Aldrich, 471305); Phosphoric acid (Sigma-Aldrich, 695017); Tetrahydrofuran, THF, inhibited (Sigma-Aldrich, 494461); Acetonitrile, HPLC grade (Sigma-Aldrich, 360457); Methanol (Sigma-Aldrich, 439193).

HPLC Mobile Phase: Mobile Phase A: 0.2% butylamine, 0.5% phosphoric acid and 1% THF in water; Mobile Phase B: 50% Mobile Phase A in 50% acetonitrile; Mobile Phase C: 40% methanol in water.

i. Equipment: Waters Alliance 2695 HPLC system, equipped with temperature control module (TCM) or column oven and model 2475 multi-wavelength fluorescence detector; Waters Empower Chromatography Software; C18 Ultrasphere ODS, reversed-phase column, 4.6 x 150mm, 5$\mu$m (Beckman 235330); Vivaspin 500 filter concentrators, 10kDa MWCO PES (Sartorius-Stedim VS0102); 2.0mL microcentrifuge tubes (low protein binding); 2-1000$\mu$L pipettes (Rainin); Dry heat block; Screw neck injection vials with caps (Waters); Eppendorf centrifuge; Vortex mixer; UV spectrophotometer; 2mm cuvette; and General laboratory supplies.

**Procedures**

Preparation of Mobile Phase and Reagents:
0.05% Acetic Acid (AcOH) Solution: Add 50$\mu$L of glacial acetic acid to 80mL of water. QS to 100mL with water. Mix well. Store at room temperature. Expires in 3 months.

0.5M Sodium Bisulfate Solution: Dissolve 6.9g of sodium bisulfate in 100mL of water. Prepare fresh.

0.25M Sodium Bisulfate Solution: Add 50mL 0.5M sodium to 50mL water. Mix well. Prepare fresh.

1.0mM Sialic Acid Stock Solution: Dissolve 7.75mg of NANA and 8.16mg of NGNA in 25mL with 0.05% AcOH solution. Store at -20°C in 1mL aliquots. Expires in 2 years.

Sialic Acid Working Solution: Thaw an aliquot of sialic acid stock solution. Add 100$\mu$L to 4.9mL of 0.25M sodium bisulfate. The working solution contains 20nmols sialic acid per mL. Prepare fresh.

O-Phenylenediamine (OPD) Derivatization Solution (20mg/mL): Add 40mg OPD to a Biostor tube. QS to 2mL with 0.25M sodium bisulfate and mix well. Enough for ~19 preparations. Prepare fresh.

HPLC Mobile Phase A: Add 4.0mL of 1-butylamine, 10mL phosphoric acid and 20mL THE to 1.8L of water in a glass bottle. QS to 2L with water and mix well. Store at room temperature. Expires in 6 months.

HPLC Mobile Phase B: Mix 0.5L HPLC Mobile Phase A and 0.5L acetonitrile in a glass bottle for a 1L total volume. Mix well. Store at room temperature. Expires in 6 months.

HPLC Mobile Phase C: Add 400mL of methanol to 600mL of water. Mix well. Store at room temperature. Expires in 6 months.

Sample Hydrolysis: Add 500$\mu$L of water to a spin filter. Centrifuge at 12000rpm for 5min to rinse. Discard filtrate. Place 4mg of sample (400pL at 10mg/mL) into the rinsed filter. QS to 500$\mu$L with 0.05 % AcOH solution. Centrifuge at 12000rpm for 8-10 min. Discard filtrate, QS to 500$\mu$L with 0.05% AcOH solution and centrifuge at 12000rpm for 8-10 min. Repeat for a total of two wash steps. Adjust the final volume to approximately 250pL with 0.05% AcOH solution. Vortex and transfer to microfuge tube. Pool replicates as needed (this is the "undiluted protein." Make a 1:10 dilution for UV concentration determination by adding 50$\mu$L of the protein to 450pL of 0.05% AcOH solution. Blank the UV detector with 0.05% AcOH solution. Measure absorbance at 280nm of each 1:10 diluted protein sample. Determine the protein concentration in mg/mL by multiplying the A280 by the dilution factor of 10 and by the absorbance factor specific to the protein with regard to cell path length. Samples may be stored at 4°C before continuing hydrolysis. Place 250pg of undiluted protein, in duplicate, in microfuge tubes. QS to 100$\mu$L with 0.5M sodium bisulfate. Place 50$\mu$L of 0.05% AcOH solution and 50$\mu$L of 0.5M sodium bisulfate in a tube as the negative control. Cap tubes tightly, mix well and incubate in a heating block at 80°C for 20 minutes. Let tubes cool to room temperature.

Prepare the standard curve in tubes by the dilution series, Table 13, using the 20nmol/mL sialic acid Working Solution (the "sialic acid standards):

**Table 13: Standard Curve Dilution Series**

| Target Conc. (nmol/mL) | 20 (Working Sol'n) | 3.0 | 2.5 | 2.0 | 1.5 | 1.0 |
|---|---|---|---|---|---|---|
| Volume of 0.25M sod. bis. (μL) | | 1445 | 233 | 220 | 200 | 133 |
| Volume of Previous Sol'n (μL) | | 255 | 1167 | 880 | 600 | 267 |
| On-column Standard Level (pmol) | | 30 | 25 | 20 | 15 | 10 |

Add 100μL of each sialic acid standard (10, 15, 20, 25 and 30pmol) to tubes.

Add 100μL of OPD solution to each of the standards, samples and negative control. Cap tubes tightly, mix and place in a heating block at 80°C for 40 minutes. Allow to cool and add 0.8mL HPLC Mobile Phase A to each tube. Mix well, centrifuge at 12000rpm for 5 minutes and transfer to autosampler vials without disturbing the pellet.

HPLC. Equilibrate column with 10% B at 1.0mL/min for 30 minutes. Running conditions are as follows:

| | |
|---|---|
| TCM or column oven: | 20 ± 5°C |
| Sample Tray Temperature: | 5 ± 2°C |
| Degasser Unit: | Normal |
| Sampler Interval: | 1.0 sec |
| Wavelength: | 230nm excitation, 425nm emission |
| Flow: | 1.0 mL/min |
| Run Time: | 38 min |

Gradient:

**Table 14**

| Time (min) | % B | Curve |
|---|---|---|
| 0.0 | 10 | 6 |
| 15.0 | 10 | 6 |
| 15.1 | 95 | 6 |
| 26.0 | 95 | 6 |
| 26.1 | 10 | 6 |
| 38.0 | 10 | 6 |

Set processing to "RUN ONLY". Inject 100μL each of Mobile Phase A blank, negative control, standard curve levels and test samples:

**Table 15**

| Inj Vol (μL) | SampleName | Sample Type | Level | Function | Method Set |
|---|---|---|---|---|---|
| 100.0 | blank- Solvent A | Unknown | | Inject Samples | Sialic Acid |
| 100.0 | 10pmol | Standard | 1 | Inject Standards | Sialic Acid |
| 100.0 | 15pmol | Standard | 2 | Inject Standards | Sialic Acid |
| 100.0 | 20pmol | Standard | 3 | Inject Standards | Sialic Acid |
| 100.0 | 25pmol | Standard | 4 | Inject Standards | Sialic Acid |
| 100.0 | 30pmol | Standard | 5 | Inject Standards | Sialic Acid |
| 100.0 | negative control | Unknown | | Inject Samples | Sialic Acid |
| 100.0 | reference replicate #1 | Unknown | | Inject Samples | Sialic Acid |
| 100.0 | reference replicate #2 | Unknown | | Inject Samples | Sialic Acid |

(continued)

| Inj Vol (μL) | SampleName | Sample Type | Level | Function | Method Set |
|---|---|---|---|---|---|
| 100.0 | sample replicate #1 | Unknown | | Inject Samples | Sialic Acid |
| 100.0 | sample replicate #2 | Unknown | | Inject Samples | Sialic Acid |

At the end of run, wash column with Mobile Phase C for 15min and store it in 100% Mobile Phase B prior to shutdown of the system.

**Data Processing and Analysis**

Clear previous calibration curves if needed, calibrate the standard curve and then process samples. Processing method amounts for standard levels are as follows:

**Table 16**

| Level | NANA | NGNA |
|---|---|---|
| 1 | 10 | 10 |
| 2 | 15 | 15 |
| 3 | 20 | 20 |
| 4 | 25 | 25 |
| 5 | 30 | 30 |

Processing parameters are as follows:

Threshold: 20pv/sec
Peak Width: 150sec
Inhibit Integration: 0.0 to 9 and 16-38min
Label peaks as follows:

**Table 17**

| Peak Name | Approximate Retention Time (min.) |
|---|---|
| NGNA | 11.3 |
| NANA | 13.2 |

Retention times may vary. Processing parameters may be adjusted, if necessary.

**Calculations:** Calculate test sample sialic acid amounts in pmols based on each calibrated standard curve. Report the sialic acid (pmol) per protein (mg) amounts for each sialic acid present within the calibrated range. The result may be converted into mmol of sialic acid per mol of protein. For values that fall below the lowest calibrated standard value of 10pmol, report results as "below LOQ".

Calculations may be adjusted as needed. Equivalent equipment and materials may be used. And the procedures may be adjusted as needed.

**Example 6. Comparison of Glycosylation Patterns Between Eculizumab That Is Cultured in NS0 cells and an Eculizumab Variant that Is Cultured in CHO cells**

CHO cells bearing a vector with the eculizumab variant consisting of the amino acid sequences of SEQ ID NO: 3 and SEQ ID NO: 4 are grown in a 200L bioreactor culture. The eculizumab variant protein is purified by standard methods and tested at 10 mg/ml by standard methods. No NGNA is detected on the protein. The neutral oligosaccharides are about 99.99% of the total oligosaccharide content of the protein.

NS0 cells bearing a vector with eculizumab consisting of the amino acid sequences of SEQ ID NO: 1 and SEQ ID NO: 2 are grown in several 200L bioreactor cultures. The eculizumab protein is purified by standard methods and tested at 10 mg/ml by standard methods. NGNA content of between 0.1 mmol/mol and 9.6 mmol/mol is detected on the protein. The neutral oligosaccharides (glycans) are about 89% of the total oligosaccharide content of the protein.

## Example 7. Eculizumab and Eculizumab Variants That Are Cultured in CHO Cells

CHO cells bearing a vector with eculizumab and CHO cells bearing an eculizumab variant consisting of the amino acid sequences of SEQ ID NO.: 3 and SEQ ID NO.:4 are grown in 200L bioreactor cultures. The proteins are purified by standard methods. These proteins have the correct molecular weight, correct N-terminal sequence, bind specifically to human C5, and otherwise behave like eculizumab or an eculizumab variant. These proteins do not have any NGNA, do not have any GalNc, and have neutral oligosaccharide content of about 99% or more of the total oligosaccharide content of each of the protein.

## Example 8. Eculizumab Cultured in NS0 Cells

Three batches of eculizumab were manufactured using Millipore (United States, US) Bovine Serum Albumin (BSA) (A batches) and Three batches of eculizumab were produced using MP Biomedical Low IgG BSA (New Zealand, N2) (B batches).

The % monomer levels determined by analytical ultracentrifugation of eculizumab B batches were comparable to the A batches, with all values in the range of 98.3 % to 99.9 %. The % monomer determined for the ultracentrifugation eculizumab B batches with a mean of 99.1 %, the three eculizumab A batches with a mean of 99.5 %. Ultracentrifugation data were acquired using the Analytical Ultracentrifugation Facility at the University of Connecticut Biotechnology Bioservices Center. This method distinguishes monomeric IgG from aggregates consisting of dimeric or larger species. This method measures continuous sedimentation coefficient distribution to determine % monomer.

The mean molecular weight for eculizumab is calculated from the primary amino acid sequences of the light and heavy chain using NIST molecular weights and isotope percentages. Upon disulfide formation, 36 free thiol groups from 36 cysteines will form 18 disulfide bonds. By assuming the pyroglutamation of both heavy chain N-termini, clipping of both heavy chain C-terminal lysine residues, and glycosylation with two G0F glycan residues at Asn298 the theoretical eculizumab antibody's major component mean molecular weight is calculated to be 147,869.70 Da.

The determined MALDI-TOF mean molecular weight value for eculizumab A and B batches and the calculated major component is within the 1 % mass accuracy of the externally calibrated MALDI-TOF. The intact molecular weight determined for the eculizumab A batches has a mean value of 148,654 Da and the B batches has a mean value of 148,531Da. The eculizumab MALDI-TOF data were acquired using WPD-PA-006. This method identifies the molecule on the basis of intact molecular weight. Test samples were solid phase extracted and mixed with sinapinic acid matrix solution and co-precipitated on the MALDI target. This dried sample was ionized with a laser into a TOF mass spectrometer, an m/z spectrum was collected and the intact mAb m/z was converted to molecular weight. Samples were tested in triplicate and the mean intact molecular weight was determined.

The determined ESI-TOF-MS intact molecular weight value for the main peak of the eculizumab B batches is comparable to the eculizumab A batches and the calculated major component is within the 100 ppm mass accuracy of the externally calibrated ESI-ToF-MS. The A batches had a mean intact molecular weight of 147,872.03Da and the B batches a mean of 147,871.98Da. The values are consistent with the calculated major component molecular weight value for eculizumab of 147,869.70 Da. No major peaks were observed beyond the 147,000-149,500 Da range. The eculizumab ESI-TOF-MS data were acquired. This method identifies the molecule on the basis of intact molecular weight. Test samples were injected onto a C4 RP-HPLC column and eluted with an aqueous:organic solvent gradient. The eluate was then electrosprayed into a ToF mass spectrometer and a spectrum from the upper half of the chromatographic peak was deconvoluted to provide the intact molecular weight.

The determined N-terminal sequences of the heavy chain and light chain for the two eculizumab batches are consistent with the known amino acid sequence for eculizumab first fifteen residues. The heavy chain was found to be blocked with a PyroQ, as expected, and was de-blocked with pyroglutamate aminopeptidase (PGAP). N-Terminal sequence data were acquired by determining the primary sequence of the protein at the N-terminus of a polypeptide chain by sequential Edman degradation and HPLC analysis.

The observed N-Linked Oligosaccharide or glycan molecular weights are comparable between the two eculizumab batches, and consistent with the theoretical glycan molecular weights.

The monosaccharide percentages determined for the two batches are comparable.

With respect to sialic acid, NANA was not detected in any of the batches. Sialic acid data were acquired by assessing the glycosylation pattern by determining the type and relative amount of the sialic acids associated with the drug molecule. The sialic acids were chemically cleaved from the antibody by incubation with sodium bisulfate then tagged with O-phenylenediamine. Samples were injected on to an RP-HPLC system with a Beckman C18 Ultrasphere column and the tagged sialic acids were detected with a fluorescence detector (230 nm excitation; 425 nm emission).

Samples were tested in duplicates and the mean of the two results was reported. For the A batches, the NGNA contents were 9.6, 9.0, 8.3, 8.9 mmol/mol; for the B batches, the NGNA contents were 6.6, 6.4, 6.4, 6.5 mmol/mol.

**Example 8. An Eculizumab Variants Cultured in CHO Cells**

CHO cells bearing a vector with an eculizumab variant consisting of the amino acid sequences of SEQ ID NO.: 3 and SEQ ID NO.:4 are grown in 200L bioreactor cultures. The antibodies are purified by standard methods to above 99% purity and formulated at about 10 mg/ml. Several such batches were produced.

The mean molecular weight for this variant is calculated from the primary amino acid sequences of the light and heavy chain using NIST molecular weights and isotope percentages. Upon disulfide formation, 36 free thiol groups from 36 cysteines will form 18 disulfide bonds. By assuming the pyroglutamation of both heavy chain N-termini, clipping of both heavy chain C-terminal lysine residues, and glycosylation with two G0F glycan residues at Asn298 the theoretical antibody's major component mean molecular weight is calculated to be 147,827.62 Da.

The determined MALDI-TOF mean molecular weight value for two batches of this eculizumab variant and the calculated major component is within the 1 % mass accuracy of the externally calibrated MALDI- TOF (148,484 Da and 148,522 Da). This method identifies the molecule on the basis of intact molecular weight. Test samples were solid phase extracted and mixed with sinapinic acid matrix solution and co-precipitated on the MALDI target. This dried sample was ionized with a N2 laser into a TOF mass spectrometer, an m/z spectrum was collected and the intact mAb m/z was converted to molecular weight. Samples were tested in triplicate and the mean intact molecular weight was determined.

The intact molecular weight were determined for two batches. The values are consistent with the calculated major component molecular weight value of 147,827.62 Da, and within the 100 ppm mass accuracy of the externally calibrated ESI-ToF-MS. No major peaks were observed beyond the 147,000-149,500 Da range. The ESI-TOF-MS method identifies the molecule on the basis of intact molecular weight. Test samples were injected onto a C4 RP-HPLC column and eluted with an aqueous:organic solvent gradient. The eluate was then electrosprayed into a ToF mass spectrometer and a spectrum from the upper half of the chromatographic peak was deconvoluted to provide the intact molecular weight.

The determined N-terminal sequences of the heavy chain and light chain are consistent with the amino acid sequence for the two batches. The heavy chain was found to be blocked with a PyroQ, as expected, and was de-blocked with pyroglutamate aminopeptidase (PGAP). N-terminal sequencing method determines the primary sequence of the protein at the N-terminus of a polypeptide chain by sequential Edman degradation and HPLC analysis.

The totals for various types of N-linked oligosaccharides are calculated: (Total G0F, G1F), Acidic, High Mannose, Neutral, Monosialylated and Disialylated. The oligosaccharide data were acquired using a method that evaluates the glycosylation pattern by identifying the N-linked oligosaccharides associated with the drug molecule on the basis of the retention time of the enzymatically released and fluorescently tagged oligosaccharides. This method provides relative abundance of each oligosaccharide species. The oligosaccharides were enzymatically cleaved from the antibody with PNGase F and tagged with anthranilic acid. Excess anthranilic acid was removed using a HILIC filtration step. Samples were injected on to a wAX-HPLC system with a Showa Denko Asahipak Amino Column and the tagged oligosaccharides were detected with a fluorescence detector; 360 nm excitation and 420 nm emission.

The monosaccharide percentages are determined for the two batches of the eculizumab variant. The monosaccharide percentages determined are for the five monosaccharides (GlcNAc, GalNAc, Galactose, Mannose, Fucose). The monosaccharide data were acquired using an assay that characterizes the glycosylation pattern by determining the monosaccharides associated with the drug molecule on the basis of the retention time of the fluorescently tagged monosaccharides. Acid hydrolysis removed the oligosaccharides from the protein and into its constituent monosaccharides. The free monosaccharides were then tagged with anthranilic acid (AA) by reductive amination. Samples were then injected on to an RP-HPLC system with a Waters Symmetry® C-18 column and the AA tagged proteins were detected with a fluorescence detector; 360 nm excitation 420 nm emission. Samples were tested in duplicate and the value reported was the mean of the two results.

The determined NANA and NGNA sialic acid content of the two eculizumab antibody variant batches were below the limit of quantitation (< 6 mmol/mol) . No NGNA was observed for either batch produced in CHO cells. The sialic acid data were acquired using a method that assesses the glycosylation pattern by determining the type and relative amount of the sialic acids associated with the drug molecule. The sialic acids were chemically cleaved from the antibody by incubation with sodium bisulfate then tagged with O- phenylenediamine. Samples were injected on to an RP-HPLC system with a Beckman C18 Ultrasphere column and the tagged sialic acids were detected with a fluorescence detector (230 nm excitation; 425nm emission). Samples were tested in duplicates and the mean of the two results was reported.

Table 18 summarizes the data for the sugar contents of the two batches of this eculizumab variant.

**Table 18 The Sugar Contents of the Two Batches of the Eculizumab Variant**

| Oligosaccharide | % | % |
|---|---|---|
| M3N2F | 0.00 | 0.00 |
| G0F-GN | 0.66 | 0.93 |
| G0F | 90.45 | 91.26 |
| G1F | 8.79 | 7.7 |
| G2F | 0.00 | 0.00 |
| Man-5 | 0.09 | 0.12 |
| aGal1 | 0.00 | 0.00 |
| Man-6 | 0.00 | 0.00 |
| aGal2 | 0.00 | 0.00 |
| Man-7 | 0.00 | 0.00 |
| aGal3 | 0.00 | 0.00 |
| SA1-1 | 0.00 | 0.00 |
| SA1-2 | 0.00 | 0.00 |
| SA1/aGal4 | 0.00 | 0.00 |
| SA1-3 | 0.00 | 0.00 |
| SA1-4 | 0.00 | 0.00 |
| SA2-1 | 0.00 | 0.00 |
| SA2-2 | 0.00 | 0.00 |
| Total G0F, G1F, G2F | 99.24 | 98.96 |
| Acidic | 0.00 | 0.00 |
| High Mannose | 0.09 | 0.12 |
| aGal | 0.00 | 0.00 |
| Neutral | 99.99 | 100.01 |
| Monosialylated | 0.00 | 0.00 |
| Disialylated | 0.00 | 0.00 |
| Monosaccharide | (nmol mono/ mg protein) | (nmol mono/ mg protein) |
| GlcNAc | 22.14 | 29.26 |
| GalNAc | 0.00 | 0.00 |
| Galactose | 0.66 | 0.82 |
| Mannose | 20.25 | 23.24 |
| Fucose | 5.38 | 6.53 |
| Total | 48 | 60 |
| % Glycosylation | 0.93 % | 1.16% |
| **Sialic Acid** | (mmol/mol) | (mmol/mol) |
| **NGNA** | ND | ND |
| **NANA** | < LoQ | < LoQ |

**Other Embodiments**

The foregoing description discloses only exemplary embodiments.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the appended claims. Thus, while only certain features of the invention have been illustrated and described, many modifications and changes will occur to those skilled in the art. It is therefore to be understood that the appended claims are intended to cover all such modifications and changes as fall within the true spirit of the invention.

**TABLE 19: NUCLEIC ACID AND AMINO ACID SEQUENCES**

**SEQ ID NO:1**

QVQLVQSGAEVKKPGASVKVSCKASGYIFSNYWIQWVRQAPGQGLEWMGEILPGSGSTEYTENFKDRVT?
TRDTSTSTVYMELSSLRSEDTAVYYCARYFFGSSPNWYFDVWGQGTLVTVSSASTKGPSVFPLAPCSRS?
SESTAALGCLVKDYFPEPVTVSWNSGALTSGVHTFPAVLQSSGLYSLSSVVTVPSSNFGTQTYTCNVDHI
PSNTKVDKTVERKCCVECPPCPAPPVAGPSVFLFPPKPKDTLMISRTPEVTCVVVDVSQEDPEVQFNWY\
DGVEVHNAKTKPREEQFNSTYRVVSVLTVLHQDWLNGKEYKCKVSNKGLPSSIEKTISKAKGQPREPQV?
TLPPSQEEMTKNQVSLTCLVKGFYPSDIAVEWESNGQPENNYKTTPPVLDSDGSFFLYSRLTVDKSRWQI
GNVFSCSVMHEALHNHYTQKSLSLSLGK

**SEQ ID NO:2**

DIQMTQSPSSLSASVGDRVTITCGASENIYGALNWYQQKPGKAPKLLIYGATNLADGVPSRFS(
SGSGTDFTLTISSLQPEDFATYYCQNVLNTPLTFGQGTKVEIKRTVAAPSVFIFPPSDEQLKS(
TASVVCLLNNFYPREAKVQWKVDNALQSGNSQESVTEQDSKDSTYSLSSTLTLSKADYEKHKV?
ACEVTHQGLSSPVTKSFNRGEC

**SEQ ID NO:3**

**heavy chain (g$_{2/4}$)** (448 amino acids)

QVQLVQSGAEVKKPGASVKVSCKASGHIFSNYWIQWVRQAPGQGLEWMGEILPGSGHTEYTENI
KDRVTMTRDTSTSTVYMELSSLRSEDTAVYYCARYFFGSSPNWYFDVWGQGTLVTVSSASTKGI
SVFPLAPCSRSTSESTAALGCLVKDYFPEPVTVSWNSGALTSGVHTFPAVLQSSGLYSLSSVV?
VPSSNFGTQTYTCNVDHKPSNTKVDKTVERKCCVECPPCPAPPVAGPSVFLFPPKPKDTLMISI
TPEVTCVVVDVSQEDPEVQFNWYVDGVEVHNAKTKPREEQFNSTYRVVSVLTVLHQDWLNGKE?
KCKVSNKGLPSSIEKTISKAKGQPREPQVYTLPPSQEEMTKNQVSLTCLVKGFYPSDIAVEWE?
NGQPENNYKTTPPVLDSDGSFFLYSRLTVDKSRWQEGNVFSCSVLHEALHSHYTQKSLSLSLGI

**SEQ ID NO:4**

**light chain: (Kappa)** (214 amino acids)

DIQMTQSPSSLSASVGDRVTITCGASENIYGALNWYQQKPGKAPKLLIYGATNLADGVPSRFS(
SGSGTDFTLTISSLQPEDFATYYCQNVLNTPLTFGQGTKVEIKRTVAAPSVFIFPPSDEQLKS(
TASVVCLLNNFYPREAKVQWKVDNALQSGNSQESVTEQDSKDSTYSLSSTLTLSKADYEKHKV?
ACEVTHQGLSSPVTKSFNRGEC

**SEQ ID NO:5**
GYIFSNYWIQ

**SEQ ID NO:6**
EILPGSGSTEYTENFKD

**SEQ ID NO:7**
YFFGSSPNWYFDV

**SEQ ID NO:8**
GASENIYGALN

**SEQ ID NO:9**
GATNLAD

**SEQ ID NO:10**
QNVLNTPLT

**SEQ ID NO:11**

QVQLVQSGAEVKKPGASVKVSCKASGYIFSNYWIQWVRQAPGQGLEWMGEILPGSGSTEYTENI
KDRVTMTRDTSTSTVYMELSSLRSEDTAVYYCARYFFGSSPNWYFDVWGQGTLVTVSS

**SEQ ID NO:12**

DIQMTQSPSSLSASVGDRVTITCGASENIYGALNWYQQKPGKAPKLLIYGATNLADGVPSRFS(
SGSGTDFTLTISSLQPEDFATYYCQNVLNTPLTFGQGTKVEIK

## ASPECTS OF THE INVENTION

1. A recombinant eculizumab protein or a recombinant eculizumab variant protein having one or more of the structural features of:

Less than 0.1 mmol/mol of N-Glycolylneuraminic acid (NGNA);

Less than 0.02 nmol/mg protein of N-acetylgalactose amine (GalNAc); or

A percentage of neutral glycans that is above about 99% of total glycans.

2. The recombinant eculizumab protein or the recombinant eculizumab variant protein of aspect 1, wherein the protein has one or more of the structural features of:

No detectable N-Glycolylneuraminic acid (NGNA);

No detectable N-acetylgalactose amine (GalNAc); or

A percentage of neutral glycans that is above about 99% of total glycans.

3. A recombinant eculizumab protein or a recombinant eculizumab variant protein produced in a Chinese Hamster Ovary ("CHO") cell bearing an expression vector capable of expressing said eculizumab protein or said eculizumab variant protein in said CHO cell.

4. The recombinant eculizumab protein or the recombinant eculizumab variant protein of aspect 3, wherein the expression vector is inducible for expressing the eculizumab protein or the eculizumab variant protein in said CHO cell.

5. The recombinant eculizumab protein or the recombinant eculizumab variant protein of aspect 3, wherein the expression vector is constitutive for expressing the eculizumab protein or the eculizumab variant protein in said CHO cell.

6. The recombinant eculizumab protein or the recombinant eculizumab variant protein of any one of aspects 3-5, having one or more of the structural features of:

Less than 0.1 mmol/mol of N-Glycolylneuraminic acid (NONA);

Less than 0.02 nmol/mg protein of N-acetylgalactose amine (GalNAc); or

A percentage of neutral glycans that is above about 99% of total glycans.

7. The recombinant eculizumab protein or the recombinant eculizumab variant protein of any one of aspects 3-6, wherein the protein has one or more of the structural features of:

No detectable N-Glycolylneuraminic acid (NGNA);

No detectable N-acetylgalactose amine (GalNAc);

or a percentage of neutral glycans that is above about 99% of total glycans.

8. The recombinant eculizumab protein or the recombinant eculizumab variant protein of any one of aspects 1-7, wherein the CHO cell is cultured in a tissue culture medium without any animal derived raw materials.

9. A Chinese Hamster Ovary ("CHO") cell bearing an expression vector capable of expressing an eculizumab protein or an eculizumab variant protein in said CHO cell.

10. The CHO cell of aspect 9, wherein the expression vector is inducible for expressing the eculizumab protein or the eculizumab variant protein in said CHO cell.

11. The CHO cell of aspect 9, wherein the expression vector is constitutive for expressing the eculizumab protein or the eculizumab variant protein in said CHO cell.

12. The CHO cell of any one of aspects 9-11, wherein the CHO cell is cultured in a tissue culture medium without any animal derived raw materials.

13. A pharmaceutical composition comprising the recombinant eculizumab protein or the recombinant eculizumab variant protein of aspect 1 or aspect 3, and a pharmaceutically acceptable carrier.

14. The pharmaceutical composition of aspect 13, wherein the composition is formulated for intravenous, intraarterial, intramuscular, intradermal, subcutaneous, or intraperitoneal administration.

15. The pharmaceutical composition of any one of aspects 13-14, wherein the concentration of the recombinant eculizumab protein or the recombinant eculizumab variant protein is at least 10 mg/mL, but less than or equal to 100 mg/mL.

16. A method for inhibiting formation of terminal complement in a biological sample, the method comprising contacting a biological sample with a therapeutic agent in an amount effective to inhibit terminal complement in the biological sample, wherein the biological sample is capable of terminal complement production in the absence of the therapeutic agent and wherein the therapeutic agent is the recombinant eculizumab protein or the recombinant eculizumab variant protein of aspect 1 or aspect 3.

17. A method of treating a patient in need of treatment with eculizumab or an eculizumab variant, comprising administering to said patient the recombinant eculizumab protein or the recombinant eculizumab variant protein of aspect 1 or aspect 3.

18. The method of aspect 17, wherein the patient is diagnosed with a complement-associated disorder.

19. The method of aspect 17 or aspect 18, wherein the patient has been diagnosed with paroxysmal nocturnal hemoglobinuria ("PNH"), atypical hemolytic uremic syndrome ("aHUS")/ or Shiga-toxin-producing *E. coli* hemolytic uremic syndrome ("STEC-HUS").

20. The method of aspect 19, wherein the complement-associated disorder is selected from the group consisting of age-related macular degeneration, graft rejection, bone marrow rejection, kidney graft rejection, skin graft rejection, heart graft rejection, lung graft rejection, liver graft rejection, rheumatoid arthritis, a pulmonary condition, ischemia-reperfusion injury, atypical hemolytic uremic syndrome, thrombotic thrombocytopenic purpura, paroxysmal nocturnal hemoglobinuria, dense deposit disease, age-related macular degeneration, spontaneous fetal loss, Pauci-immune vasculitis, epidermolysis bullosa, recurrent fetal loss, multiple sclerosis, traumatic brain injury, myasthenia gravis, cold agglutinin disease, dermatomyositis, Degos' disease. Graves' disease, Hashimoto's thyroiditis, type I diabetes, psoriasis, pemphigus, autoimmune hemolytic anemia, idiopathic thrombocytopenic purpura, Goodpasture syndrome, multifocal motor neuropathy, neuromyelitis optica, antiphospholipid syndrome, sepsis. Hemorrhagic fever, and catastrophic antiphospholipid syndrome.

## Claims

1. A recombinant eculizumab protein or a recombinant eculizumab variant protein having one or more of the structural features of:

Less than 0.1 mmol/mol of N-Glycolylneuraminic acid (NGNA);
Less than 0.02 nmol/mg protein of N-acetylgalactose amine (GalNAc);
or
A percentage of neutral glycans that is above about 99% of total glycans.

2. The recombinant eculizumab protein or the recombinant eculizumab variant protein of claim 1, wherein the protein has one or more of the structural features of:

No detectable N-Glycolylneuraminic acid (NGNA);
No detectable N-acetylgalactose amine (GalNAc);
or
A percentage of neutral glycans that is above about 99% of total glycans.

3. A recombinant eculizumab protein or a recombinant eculizumab variant protein produced in a Chinese Hamster Ovary ("CHO") cell bearing an expression vector capable of expressing said eculizumab protein or said eculizumab variant protein in said CHO cell.

4. The recombinant eculizumab protein or the recombinant eculizumab variant protein of claim 3, wherein

(a) the expression vector is inducible for expressing the eculizumab protein or the eculizumab variant protein in said CHO cell; or
(b) the expression vector is constitutive for expressing the eculizumab protein or the eculizumab variant protein in said CHO cell.

5. The recombinant eculizumab protein or the recombinant eculizumab variant protein of claim 3 or claim 4, having one or more of the structural features of:

Less than 0.1 mmol/mol of N-Glycolylneuraminic acid (NONA);
Less than 0.02 nmol/mg protein of N-acetylgalactose amine (GalNAc);
or
A percentage of neutral glycans that is above about 99% of total glycans.

6. The recombinant eculizumab protein or the recombinant eculizumab variant protein of any one of claims 3-5, wherein the protein has one or more of the structural features of:

No detectable N-Glycolylneuraminic acid (NGNA);
No detectable N-acetylgalactose amine (GalNAc);
or a percentage of neutral glycans that is above about 99% of total glycans.

7. The recombinant eculizumab protein or the recombinant eculizumab variant protein of any one of claims 1-6, wherein the CHO cell is cultured in a tissue culture medium without any animal derived raw materials.

8. A Chinese Hamster Ovary ("CHO") cell bearing an expression vector capable of expressing an eculizumab protein or an eculizumab variant protein in said CHO cell.

9. The CHO cell of claim 8, wherein the expression vector is

(a) inducible for expressing the eculizumab protein or the eculizumab variant protein in said CHO cell;
(b) constitutive for expressing the eculizumab protein or the eculizumab variant protein in said CHO cell; and/or
(c) the CHO cell is cultured in a tissue culture medium without any animal derived raw materials.

10. A pharmaceutical composition comprising the recombinant eculizumab protein or the recombinant eculizumab variant protein of any one of claims 1 to 7, and a pharmaceutically acceptable carrier.

11. The pharmaceutical composition of claim 10, wherein

(a) the composition is formulated for intravenous, intraarterial, intramuscular, intradermal, subcutaneous, or intraperitoneal administration; and/or
(b) the concentration of the recombinant eculizumab protein or the recombinant eculizumab variant protein is at least 10 mg/mL, but less than or equal to 100 mg/mL.

12. A method for inhibiting formation of terminal complement in a biological sample, the method comprising contacting a biological sample with a therapeutic agent in an amount effective to inhibit terminal complement in the biological sample, wherein the biological sample is capable of terminal complement production in the absence of the therapeutic agent and wherein the therapeutic agent is the recombinant eculizumab protein or the recombinant eculizumab variant protein of any one of claims 1 to 7.

13. The recombinant eculizumab protein or the recombinant eculizumab variant protein of any one of claims 1 to 7, for use in a method of treating a patient in need of treatment with eculizumab or an eculizumab variant.

14. The method of claim 13, wherein the patient is diagnosed with a complement-associated disorder, optionally wherein the complement-associated disorder is selected from the group consisting of age-related macular degeneration, graft rejection, bone marrow rejection, kidney graft rejection, skin graft rejection, heart graft rejection, lung graft rejection, liver graft rejection, rheumatoid arthritis, a pulmonary condition, ischemia- reperfusion injury, atypical hemolytic uremic syndrome, thrombotic thrombocytopenic purpura, paroxysmal nocturnal hemoglobinuria, dense deposit disease, age-related macular degeneration, spontaneous fetal loss, Pauci-immune vasculitis, epidermolysis bullosa, recurrent fetal loss, multiple sclerosis, traumatic brain injury, myasthenia gravis, cold agglutinin disease, dermatomyositis, Degos' disease, Graves' disease, Hashimoto's thyroiditis, type I diabetes, psoriasis, pemphigus, autoimmune hemolytic anemia, idiopathic thrombocytopenic purpura, Goodpasture syndrome, multifocal motor neuropathy, neuromyelitis optica, antiphospholipid syndrome, sepsis, Hemorrhagic fever, and catastrophic antiphospholipid syndrome.

15. The method of claim 13 or claim 14, wherein the patient has been diagnosed with paroxysmal nocturnal hemoglobinuria ("PNH"), atypical hemolytic uremic syndrome ("aHUS")/ or Shiga-toxin- producing E. *coli* hemolytic uremic syndrome ("STEC-HUS").

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61856930
- US 61775060
- US 20120237515 A
- US 6355245 B
- US 9079949 B
- US 6897290 B
- US 2005265995
- WO 9627011 A
- US 4676980 A
- US 5534254 A

- US 7112324 B
- US 5525491 A
- US 5258498 A
- WO 08024188 A
- WO 07024715 A
- US 20070004909
- WO 2012135345 A
- US 7056897 B
- US 7390786 B

**Non-patent literature cited in the description**

- New Clinical Trial Data Show Substantial Improvement with Eculizumab (Soliris®) in Patients with STEC-HUS. *Alexion press release,* 03 November 2012
- **JAYAPAL K. P., WLASCHIN K. F., YAP M. G. S., HU W-S.** Recombinant protein therapeutics from CHO cells - 20 years and counting. *Chem. Eng. Prog.,* 2007, vol. 103 (10), 40-47
- **NORTH et al.** *J. Biol Chem,* 19 February 2010, vol. 285 (8), 5759-5775
- **HILLMEN et al.** *N Engl J Med,* 2004, vol. 350, 552-9
- **ROTHER et al.** *Nature Biotechnology,* 2007, vol. 25 (11), 1256-1264
- **HILLMEN et al.** *N Engl J Med,* 2006, vol. 355 (12), 1233-1243
- **ZUBER et al.** *Nature Reviews Nephrology,* 2012, vol. 8, 643-657
- **NORIS et al.** *Nat Rev Nephrol.,* 18 September 2012, vol. 8 (11), 622-33
- **HILLMEN et al.** *N Engl J Med,* 2004, vol. 350 (6), 552-9
- **SONG et al.** *J Clin Invest,* 2003, vol. 11 (12), 1875-1885
- **SURESH et al.** *Methods in Enzymology,* 1986, vol. 121, 210
- **BRENNAN et al.** *Science,* 1985, vol. 229, 81
- **SHALABY et al.** *J. Exp. Med.,* 1992, vol. 175, 217-225
- **KOSTELNY et al.** *J Immunol,* 1992, vol. 148 (5), 1547-1553
- **HOLLINGER et al.** *Proc Natl Acad Sci USA,* 1993, vol. 90, 6444-6448
- **GRUBER et al.** *J Immunol,* 1994, vol. 152, 5368

- **TUTT et al.** *J Immunol,* 1991, vol. 147, 60
- **SEGAL ; BAST.** *Curr Protocols Immunol,* 1995, vol. 14, 2.13.1-2.13.16
- **REN-HEIDENREICH et al.** *Cancer,* 2004, vol. 100, 1095-1103
- **KORN et al.** *J Gene Med,* 2004, vol. 6, 642-651
- **GROSSE-HOVEST et al.** *Proc Natl Acad Sci USA,* 2004, vol. 101, 6858-6863
- **MALETZ et al.** *Int J Cancer,* 2001, vol. 93, 409-416
- **HAYDEN et al.** *Ther Immunol,* 1994, vol. 1, 3-15
- **HONEMANN et al.** *Leukemia,* 2004, vol. 18, 636-644
- **ZAPATA et al.** *Protein Eng.,* 1995, vol. 8 (10), 1057-1062
- **ZHU et al.** *Biotechnology,* 1996, vol. 14, 192-196
- **HELFRICH et al.** *Int J Cancer,* 1998, vol. 76, 232-239
- **BRÜSSELBACH et al.** *Tumor Targeting,* 1999, vol. 4, 115-123
- **KIPRIYANOV et al.** *J Mol Biol,* 1999, vol. 293, 41-56
- **NETTLEBECK et al.** *Mol Ther,* 2001, vol. 3, 882-891
- **WU et al.** *Nat Biotechnol,* 2007, vol. 25 (11), 1290-1297
- **MOONGKARNDI et al.** *Immunobiol,* 1982, vol. 162, 397
- **MOONGKARNDI et al.** *Immunobiol,* 1983, vol. 165, 323
- **WARD ; ZVAIFLER.** *J Clin Invest,* 1971, vol. 50 (3), 606-16

- **WURZNER et al.** *Complement Inflamm,* 1991, vol. 8, 328-340
- Experimental Immunochemistry. CC Thomas, 1961, vol. 135-240, 135-139
- **HILLMEN et al.** *N Engl J Med,* 2004, vol. 350 (6), 552
- **SCHOCKETT et al.** *Proc Natl Acad Sci USA,* 1996, vol. 93, 5173-5176
- Current Protocols in Molecular Biology. Wiley & Sons
- Molecular Cloning--A Laboratory Manual. Cold Spring Harbor Laboratory Press, 2001
- **HARLOW ; LANE.** Antibodies: A Laboratory Manual. Cold Spring Harbor Laboratory Press, 1988
- **BENNY K. C. LO.** Antibody Engineering: Methods and Protocols. Humana Press, 2004
- **BORREBAEK.** Antibody Engineering, A Practical Guide. W.H. Freeman and Co, 1992
- **BORREBAEK.** Antibody Engineering. Oxford University Press, 1995
- **JOHNE et al.** *J Immunol Meth,* 1993, vol. 160, 191-198
- **JONSSON et al.** *Ann Biol Clin,* 1993, vol. 51, 19-26
- **JONSSON et al.** *Biotechniques,* 1991, vol. 11, 620-627
- **ISENMAN et al.** *J Immunol,* 1980, vol. 124 (1), 326-31
- **THOMAS et al.** *Mol Immunol,* 1996, vol. 33 (17-18), 1389-401
- **EVANS et al.** *Mol Immunol,* 1995, vol. 32 (16), 1183-95
- **BERGE et al.** *J Pharm Sci,* 1977, vol. 66, 1-19
- Protein Formulation and Delivery. **MCNALLY ; HASTEDT.** Drugs and the Pharmaceutical Sciences. CRC Press, 2007, vol. 175
- **BANGA.** Therapeutic peptides and proteins: formulation, processing, and delivery systems. CRC Press, 1995
- **GRANGER et al.** *Circulation,* 2003, vol. 108, 1184
- **HAVERICH et al.** *Ann Thorac Surg,* 2006, vol. 82, 486-492
- **TESTA et al.** *J Thorac Cardiovasc Surg,* 2008, vol. 136 (4), 884-893